(19) Europäisches Patentamt / European Patent Office / Office européen des brevets

(11) **EP 2 149 327 A1**

(12) **EUROPEAN PATENT APPLICATION**
published in accordance with Art. 153(4) EPC

(43) Date of publication:
**03.02.2010 Bulletin 2010/05**

(51) Int Cl.:
**A61B 1/00** *(2006.01)*          **G01B 7/00** *(2006.01)*

(21) Application number: **08752052.4**

(22) Date of filing: **24.04.2008**

(86) International application number:
**PCT/JP2008/057961**

(87) International publication number:
**WO 2008/142951 (27.11.2008 Gazette 2008/48)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **21.05.2007 JP 2007134643**

(71) Applicant: **Olympus Corporation**
**Tokyo 151-0072 (JP)**

(72) Inventors:
• **UCHIYAMA, Akio**
**Tokyo 151-0072 (JP)**
• **SATO, Ryoji**
**Tokyo 151-0072 (JP)**
• **KIMURA, Atsushi**
**Tokyo 151-0072 (JP)**
• **KARASAWA, Ryo**
**Tokyo 151-0072 (JP)**

(74) Representative: **Schmidt, Steffen J. et al**
**Wuesthoff & Wuesthoff**
**Patent- und Rechtsanwälte**
**Schweigerstrasse 2**
**81541 München (DE)**

(54) **POSITION DETECTING SYSTEM AND POSITION DETECTING METHOD**

(57) The position or the direction of a first marker which produces an alternating magnetic field by means of an external power supply is detected precisely even if the first marker coexists with a second marker which includes a resonance circuit having a resonance frequency the same as or close to the frequency of the alternating magnetic field. There is provided a position detection system (1) including a first marker (4) that produces a first alternating magnetic field having a single set of first position-calculating frequencies that are a predetermined frequency away from each other; a second marker (3) provided with a magnetic induction coil (5) having as a resonance frequency a substantially central frequency interposed between the single set of first position-calculating frequencies; a magnetic-field detection section (13) that is disposed outside the working region and that detects a magnetic field at the first position-calculating frequencies; an extracting section (30) that extracts from the detected magnetic field the sum of the intensities of a single set of first detection-magnetic-field components having the single set of first position-calculating frequencies; and a position/direction analyzing section (22) that calculates the position or the direction of the first marker (4) based on the extracted sum.

FIG. 1

**Description**

Technical Field

The present invention relates to a position detection system and a position detection method.

Background Art

Position detection apparatuses that detect the position of a marker inserted into a body cavity by causing the marker to produce an alternating magnetic field by means of an external power supply and then detecting, outside the body, the alternating magnetic field produced by the marker are conventionally known (e.g., refer to Patent Document 1). Furthermore, position detection systems for capsule medical devices that detect the position and the direction of a capsule medical device delivered into the body of a subject by externally applying a position-detecting magnetic field and detecting the absolute-value intensity of an induced magnetic field produced in a magnetic induction coil disposed in the capsule medical device are also well known (e.g., refer to Non-patent Document 1).
Patent Document 1: Japanese Unexamined Patent Application, Publication No. 2000-81303
Non-patent Document 1: Tokunaga plus seven other authors, Precision Position-detecting System Using an LC Resonant Magnetic Marker. Journal of the Magnetics Society of Japan 2005; Vol. 29, No. 2:153-156

Disclosure of Invention

However, if a first marker which produces an alternating magnetic field by means of an external power supply coexists with a second marker which includes a resonance circuit having a resonance frequency in the proximity of the frequency of that alternating magnetic field, then an induced magnetic field is produced from the resonance circuit of the second marker due to the alternating magnetic field produced by the first marker. As a result, because merely detecting the absolute-value intensity of the magnetic field at the frequency of the alternating magnetic field involves simultaneous detection of the induced magnetic field, the magnetic-field intensity obtained in this case differs from the magnetic-field intensity obtained in a case where the alternating magnetic field alone is detected. For this reason, it has been difficult to precisely calculate the position or the direction of the first marker.
An object of the present invention is to provide a position detection system and a position detection method capable of precisely detecting the position or the direction of a first marker which produces an alternating magnetic field by means of an external power supply even if the first marker coexists with a second marker which includes a resonance circuit having a resonance frequency the same as or close to the frequency of the alternating magnetic field.
To achieve the above-described object, the present invention provides the following solutions.
A first aspect of the present invention is a position detection system including a first marker that produces, by means of an external power supply, a first alternating magnetic field having a single set of first position-calculating frequencies that are a predetermined frequency away from each other; a second marker including a magnetic induction coil having as a resonance frequency a substantially central frequency interposed between the single set of first position-calculating frequencies; a magnetic-field detection section that is disposed outside a working region of the second marker and that detects a magnetic field at the first position-calculating frequencies; an extracting section that extracts from the magnetic field detected by the magnetic-field detection section the sum of intensities of a single set of first detection-magnetic-field components having the single set of first position-calculating frequencies; and a position/direction analyzing section that calculates at least one of a position and a direction of the first marker based on the extracted sum.
According to the first aspect of the present invention, the first alternating magnetic field, having a single set of first position-calculating frequencies that are a predetermined frequency away from each other, produced from the first marker by means of an external power supply is received by the magnetic induction coil mounted in the second marker. In response to this first alternating magnetic field, the magnetic induction coil may produce an induced magnetic field (hereinafter, referred to as the induced magnetic field associated with the first alternating magnetic field), depending on the resonance characteristics. In this case, at the single set of first position-calculating frequencies, the magnetic-field detection section detects a magnetic field where the first alternating magnetic field coexists with the induced magnetic field associated with the first alternating magnetic field.
Like the first alternating magnetic field, the induced magnetic field associated with the first alternating magnetic field has a single set of first position-calculating frequencies. On the other hand, because the first detection-magnetic-field components are magnetic-field components having the single set of first position-calculating frequencies, they contain information about the induced magnetic field, in addition to information about the first alternating magnetic field, when the induced magnetic field associated with the first alternating magnetic field is produced. Furthermore, because the resonance frequency of the magnetic induction coil is a substantially central frequency interposed between the single set of first position-calculating frequencies, the induced magnetic fields associated with the first alternating magnetic field have the characteristic that they differ from each other in the magnitude relationship of intensity with respect to the first alternating magnetic field and that they have substantially the same absolute value of intensity.

Therefore, when the sum of the intensities of the single set of first detection-magnetic-field components is calculated through the operation of the extracting section, the items of information about the induced magnetic field associated with the first alternating magnetic field are canceled out, and therefore, only the information about the first alternating magnetic field can be extracted from the magnetic field detected by the magnetic-field detection section. Because of this, the position/direction analyzing section can calculate at least one of the position and the direction of the first marker using only the intensity information of the first alternating magnetic field produced from the first marker. As a result, even if the first marker, which produces a magnetic field by means of the external power supply, coexists with the second marker having the magnetic induction coil, the position or the direction of the first marker can be calculated with high precision without being affected by the induced magnetic field.

The above-described first aspect may be configured such that the single set of first position-calculating frequencies may be frequencies near the resonance frequency, the extracting section may extract the difference between the intensities of the single set of first detection-magnetic-field components from the magnetic field detected by the magnetic-field detection section; and the position/direction analyzing section may calculate at least one of a position and a direction of the second marker based on the difference between the intensities.

By doing so, the position/direction analyzing section not only calculates at least one of the position and the direction of the first marker based on the sum extracted by the extracting section but also calculates at least one of the position and the direction of the second marker based on the intensity of the extracted difference.

Here, because the single set of first position-calculating frequencies are frequencies near the resonance frequency, the magnetic induction coil produces an induced magnetic field in response to the first alternating magnetic field. Furthermore, as described above, the induced magnetic fields associated with the first alternating magnetic field have the characteristic that they differ from each other in the magnitude relationship of intensity with respect to the first alternating magnetic field at the single set of first position-calculating frequencies.

On the other hand, because the first detection-magnetic-field components are magnetic-field components having the single set of first position-calculating frequencies, they contain information about the first alternating magnetic field and information about the induced magnetic field associated with the first alternating magnetic field. Hence, when the difference between the intensities of the single set of first detection-magnetic-field components is calculated through the operation of the extracting section, the items of information about the first alternating magnetic field are canceled out, and therefore only the information about the induced magnetic field associated with the first alternating magnetic field can be extracted from the magnetic field detected by the magnetic-field detection section.

By doing so, the position/direction analyzing section can calculate at least one of the position and the direction of the second marker using the intensity information of the induced magnetic field produced from the second marker. As a result, even if the first marker, which produces a magnetic field by means of the external power supply, coexists with the second marker having the magnetic induction coil, at least one of the position and the direction of both the first marker and the second marker can be calculated with high precision.

Furthermore, in the above-described structure, a magnetic-field generating unit that is disposed outside the working region of the second marker and that produces a second alternating magnetic field having the single set of first position-calculating frequencies may be provided, and the single set of first detection-magnetic-field components may be the difference between a magnetic field having the first position-calculating frequencies detected when the first alternating magnetic field is produced and a magnetic field having the first position-calculating frequencies detected before the first alternating magnetic field is produced.

By doing so, because the second alternating magnetic field, which is produced by the magnetic-field generating unit disposed outside the working region of the second marker, has the same frequency as that of the above-described first alternating magnetic field, the magnetic induction coil produces induced magnetic fields in response to the first alternating magnetic field and the second alternating magnetic field (hereinafter, referred to as the induced magnetic fields associated with the first and second alternating magnetic fields). The magnetic-field detection section detects a magnetic field where the first alternating magnetic field, the second alternating magnetic field, and the induced magnetic field are mixed at the first position-calculating frequencies.

Here, the magnetic field that is detected at the first position-calculating frequencies when the first and second alternating magnetic fields are produced contains information about the first alternating magnetic field, the second alternating magnetic field, and the induced magnetic fields associated with the first and second alternating magnetic fields. On the other hand, when only the second alternating magnetic field is produced, the magnetic induction coil produces an induced magnetic field in response to the second alternating magnetic field (hereinafter, referred to as the induced magnetic field associated with the second alternating magnetic field). At this time, the magnetic field detected at the first position-calculating frequencies contains information about the second alternating magnetic field and the induced magnetic field associated with the second alternating magnetic field.

Therefore, assuming that the difference of magnetic-field information between when and before the first alternating magnetic field is produced is the first detection-magnetic-field components, the first detection-magnetic-field component at each frequency contains only information about the first alternating magnetic field and information about the induced magnetic field associated with the first alternating magnetic field.

For this reason, when the sum of the intensities of the single set of first detection-magnetic-field components is calculated through the operation of the extracting section, the items of information about the induced magnetic field associated with the first alternating magnetic field are canceled out, and therefore, only information about the intensity of the first alternating magnetic field can be extracted from the magnetic field detected by the magnetic-field detection section.

Furthermore, the difference between the intensities of the single set of first detection-magnetic-field components does not contain information about the first alternating magnetic field or the second alternating magnetic field, for the same reason as described above, but contains only information about the induced magnetic fields associated with the first and second alternating magnetic fields.

Therefore, when the difference between the intensities of the single set of first detection-magnetic-field components is calculated through the operation of the extracting section, only the information about the induced magnetic fields associated with the first and second alternating magnetic fields can be extracted.

Because of this, the position/direction analyzing section can calculate at least one of the position and the direction of the first marker using only the information about the intensity of the first alternating magnetic field and also can calculate at least one of the position and the direction of the second marker using the intensity information of the induced magnetic field produced from the second marker.

As a result, even if the first marker, which produces a magnetic field by means of the external power supply, coexists with the second marker having the magnetic induction coil, at least one of the position and the direction of both the first marker and the second marker can be calculated with high precision. Furthermore, because not only the first alternating magnetic field but also the second alternating magnetic field produces an induced magnetic field from the second marker, the intensity of the induced magnetic field can be increased.

Furthermore, in the above-described first aspect, a magnetic-field generating unit that is disposed outside the working region of the second marker and that produces a second alternating magnetic field having a single set of second position-calculating frequencies that are near the resonance frequency, that differ from the first position-calculating frequencies, and that are a predetermined frequency away from the resonance frequency, with the second position-calculating frequencies being on either side of the resonance frequency, may be provided, and the magnetic-field detection section may detect a magnetic field at the second position-calculating frequencies, the extracting section may extract the difference between intensities of a single set of second detection-magnetic-field components having the single set of second position-calculating frequencies from the magnetic field detected by the magnetic-field detection section, and the position/direction analyzing section may calculate at least one of a position and a direction of the second marker based on the difference between the intensities.

By doing so, because the single set of second position-calculating frequencies of the second alternating magnetic field produced by the magnetic-field generating unit disposed outside the working region of the second marker are frequencies near the resonance frequency, the magnetic induction coil produces the induced magnetic field associated with the first alternating magnetic field in response to the first alternating magnetic field and produces an induced magnetic field having the single set of second position-calculating frequencies in response to the second alternating magnetic field (the induced magnetic field associated with the second alternating magnetic field). The magnetic-field detection section detects, at the single set of first position-calculating frequencies, a magnetic field where the first alternating magnetic field coexists with the induced magnetic field associated with the first alternating magnetic field and detects, at the single set of second position-calculating frequencies, a magnetic field where the second alternating magnetic field coexists with the induced magnetic field associated with the second alternating magnetic field.

Then, through the operation of the extracting section, not only is the sum of the intensities of the single set of first detection-magnetic-field components extracted but also the difference between the intensities of the single set of second detection-magnetic-field components is extracted from the magnetic field detected by the magnetic-field detection section. Furthermore, through the operation of the position/direction analyzing section, at least one of the position and the direction of the first marker is calculated based on the sum extracted by the extracting section, and at least one of the position and the direction of the second marker is calculated based on the intensity of the extracted difference.

In this case, for the same reason as described above, the induced magnetic fields associated with the second alternating magnetic field have the characteristic that they differ from each other in the magnitude relationship of intensity with respect to the second alternating magnetic field at the single set of second position-calculating frequencies. On the other hand, because the second detection-magnetic-field components are magnetic-field components having the single set of second position-calculating frequencies, they contain information about the second alternating magnetic field and information about the induced magnetic field associated with the second alternating magnetic field. Therefore, when the difference between the intensities of the single set of second detection-magnetic-field components is calculated through the operation of the extracting section, the items of information about the second alternating magnetic field are canceled out, and therefore, only the information about the induced magnetic field associated with the second alternating magnetic field can be extracted from the magnetic field detected by the magnetic-field detection section.

By doing so, the position/direction analyzing section can calculate at least one of the position and the direction of the second marker using intensity information of the induced magnetic field produced from the second marker. As a result, even if the first marker, which produces a magnetic field by means of the external power supply, coexists with the second marker having the magnetic induction coil, at least one of the position and the direction of both the first marker and the second marker can be calculated with high precision.

Furthermore, in the above-described first aspect, a magnetic-field generating unit that is disposed outside the working region of the second marker and that produces a second alternating magnetic field having the resonance frequency may be provided, and the magnetic-field detection section may detect a magnetic field at the resonance frequency, the extracting section may extract from the magnetic field detected by the magnetic-field detection section a second detection-magnetic-field component that has the resonance frequency and that has a phase shifted by n/2 relative to the phase of the second alternating magnetic field, and the position/direction analyzing section may calculate at least one of a position and a direction of the second marker based on an intensity of the second detection-magnetic-field component.

By doing so, the magnetic-field generating unit disposed outside the working region of the second marker produces the second alternating magnetic field having the resonance frequency of the magnetic induction coil mounted in the second marker. The magnetic induction coil produces the induced magnetic field associated with the first alternating magnetic field in response to the first alternating magnetic field and produces the induced magnetic field associated with the second alternating magnetic field in response to the second alternating magnetic field. The magnetic-field detection section detects, at the single set of first position-calculating frequencies, a magnetic field where the first alternating magnetic field coexists with the induced magnetic field associated with the first alternating magnetic field and detects, at the resonance frequency, a magnetic field where the second alternating magnetic field coexists with the induced magnetic field associated with the second alternating magnetic field.

The extracting section extracts the sum of the intensities of the single set of first detection-magnetic-field components and extracts the second detection-magnetic-field component from the magnetic field detected by the magnetic-field detection section. The position/direction analyzing section not only calculates at least one of the position and the direction of the first marker based on the sum extracted by the extracting section but also calculates at least one of the position and the direction of the second marker based on the intensity of the extracted second detection-magnetic-field component.

Here, the induced magnetic field associated with the second alternating magnetic field has the same frequency as and a phase shifted by n/2 relative to that of the second alternating magnetic field. On the other hand, because the second detection-magnetic-field component is a magnetic-field component that has the same frequency as and a phase shifted by n/2 relative to that of the second alternating magnetic field, it does not contain information about the second alternating magnetic field but contains only the information about the induced magnetic field associated with the second alternating magnetic field. Therefore, when the second detection-magnetic-field component is extracted through the operation of the extracting section, only the information about the induced magnetic field associated with the second alternating magnetic field can be extracted from the magnetic field detected by the magnetic-field detection section.

By doing so, the position/direction analyzing section can calculate at least one of the position and the direction of the second marker using only the intensity information of the induced magnetic field produced from the second marker. As a result, even if the first marker, which produces a magnetic field by means of the external power supply, coexists with the second marker having the magnetic induction coil, at least one of the position and the direction of both the first marker and the second marker can be calculated with high precision.

Furthermore, in any of the above-described position detection systems, a resonance circuit including the magnetic induction coil may satisfy the following relation at the first position-calculating frequencies.

[Expression 1]

$$\frac{-(L + \frac{1}{\omega_1^2 C})(\omega L - \frac{1}{\omega_1 C})}{\sqrt{R^2 - (\omega_1 L - \frac{1}{\omega_1 C})^2}} = \frac{-(L + \frac{1}{\omega_2^2 C})(\omega L - \frac{1}{\omega_2 C})}{\sqrt{R^2 - (\omega_2 L - \frac{1}{\omega_2 C})^2}}$$

where $\omega_1 = 2\pi f_1$, $\omega_2 = 2\pi f_2$, and $\omega_1 < \omega_0 = 2\pi f_0 < \omega_2$ ($f_0$: resonance frequency).

By doing so, the detection intensities of the induced magnetic field associated with the first alternating magnetic field, as detected by the same sense coils at each frequency, can be made equal. As a result, through a simple addition operation involving the intensities of the single set of first detection-magnetic-field components, only the information about the first alternating magnetic field can be extracted by canceling out the items of information about the induced magnetic field.

Furthermore, in any of the above-described position detection systems, a plurality of the first markers may be provided, and a plurality of the first position-calculating frequencies may differ from one another.

By doing so, a plurality of first markers can be identified.

Furthermore, in any of the above-described position detection systems, the first marker may be provided at a front end portion of an endoscope.

Furthermore, if a plurality of the above-described first markers are provided as described above, the plurality of first markers may be provided along a longitudinal direction of an inserting section of an endoscope.

Furthermore, in any of the above-described position detection systems, the second marker may be provided in a capsule medical device.

Furthermore, any of the above-described position detection systems where the position/direction analyzing section calculates at least one of the position and the direction of the second marker may include a magnetic-field acting section in the second marker; a propulsion-magnetic-field generating unit that produces a propulsion magnetic field acting upon the magnetic-field acting section; and a propulsion-magnetic-field control section that controls an intensity and a direction of the propulsion magnetic field based on at least one of the position and the direction of the second marker calculated by the position/direction analyzing section.

By doing so, the intensity and direction of the propulsion magnetic field, which has been produced by the propulsion-magnetic-field generating unit and is made to act upon the magnetic-field acting section of the second marker, is controlled through the operation of the propulsion-magnetic-field control section based on at least one of the position and the direction of the second marker calculated by the position/direction analyzing section. Because of this, the propulsion of the second marker can be controlled based on the position or the direction of the second marker.

Furthermore, a second aspect according to the present invention is a position detection method including a magnetic-field generating step of causing a first marker to produce, by means of an external power supply, a first alternating magnetic field having a single set of first position-calculating frequencies that are a predetermined frequency away from each other; an induction magnetic-field generating step of causing a second marker having a magnetic induction coil to produce an induced magnetic field in response to the first alternating magnetic field; a magnetic-field detecting step of detecting a magnetic field at the first position-calculating frequencies; an extracting step of extracting from the detected magnetic field the sum of intensities of a single set of first detection-magnetic-field components having the single set of first position-calculating frequencies; and a position/direction analyzing step of calculating at least one of a position and a direction of the first marker based on the extracted sum.

The above-described second aspect may be configures such that the extracting step may include the step of extracting the difference between the intensities of the single set of first detection-magnetic-field components from the detected magnetic field, and the position/direction analyzing step may include the step of calculating at least one of a position and a direction of the second marker based on the extracted difference between the intensities.

Furthermore, in the above-described structure, the magnetic-field generating step may include the step of producing a second alternating magnetic field having the single set of first position-calculating frequencies, the induction magnetic-field generating step may include the step of causing the second marker to produce an induced magnetic field in response to the second alternating magnetic field, and the single set of detection-magnetic-field components may be the difference between a magnetic field having the first position-calculating frequencies detected when the first alternating magnetic field is produced and a magnetic field having the first position-calculating frequencies detected before the first alternating magnetic field is produced.

Furthermore, in the above-described second aspect, the magnetic-field generating step may include the step of producing a second alternating magnetic field having a single set of second position-calculating frequencies near the single set of first position-calculating frequencies, the magnetic-field detecting step may include the step of detecting a magnetic field at the second position-calculating frequencies, the extracting step may include the step of extracting from the detected magnetic field the difference between intensities of a single set of second detection-magnetic-field components having the single set of second position-calculating frequencies, and the position/direction analyzing step may include the step of calculating at least one of a position and a direction of the second marker based on the extracted difference between the intensities.

Furthermore, in the above-described second aspect, the magnetic-field generating step may include the step of producing a second alternating magnetic field having the resonance frequency, the magnetic-field detecting step may include the step of detecting a magnetic field at the resonance frequency, the extracting step may include the step of extracting from the detected magnetic field a second detection-magnetic-field component that has the resonance frequency and that has a phase shifted by n/2 relative to the phase of the second alternating magnetic field, and the position/direction analyzing step may calculate at least one of a position and a direction of the second marker based on an intensity of the extracted second detection-magnetic-field component.

According to the position detection system and position detection method of the present invention, an advantage is afforded in that even if a first marker that produces an alternating magnetic field by means of an external power supply coexists with a second marker provided with a resonance circuit having a resonance frequency that is the same as or near the frequency of the alternating magnetic field, the position or the direction of the first marker can be detected precisely.

Brief Description of Drawings

[Fig. 1] Fig. 1 is a block diagram showing the overall structure of a position detection system according to a first embodiment of the present invention.

[Fig. 2] Fig. 2 is a block diagram showing the detailed structure of the position detection system in Fig. 1.

[Fig. 3] Fig. 3 is a flowchart illustrating waveform generation by a position detection method using the position detection system in Fig. 1.

[Fig. 4] Fig. 4 is a flowchart illustrating the first half of actual measurement by the position detection method in Fig. 3.

[Fig. 5] Fig. 5 is a flowchart continued from the actual measurement in Fig. 4.

[Fig. 6] Fig. 6 is a flowchart continued from the actual measurement in Fig. 5.

[Fig. 7] Fig. 7 is an overall structural diagram depicting a medical-device guidance system provided with a position detection system according to a second embodiment of the present invention.

[Fig. 8] Fig. 8 is a longitudinal sectional view showing one example of a capsule medical device used with the medical-device guidance system in Fig. 7.

[Fig. 9] Fig. 9 is a block diagram depicting an overall structure of the position detection system according to this embodiment provided in the medical-device guidance system of Fig. 7.

[Fig. 10] Fig. 10 is a block diagram depicting the detailed structure of the position detection system in Fig. 9.

[Fig. 11] Fig. 11 is a flowchart illustrating calibration by a position detection method using the position detection system in Fig. 9.

[Fig. 12] Fig. 12 is a flowchart illustrating the first half of actual measurement by the position detection method in Fig. 11.

[Fig. 13] Fig. 13 is a flowchart continued from the actual measurement in Fig. 12.

[Fig. 14] Fig. 14 is a flowchart continued from the actual measurement in Fig. 13.

[Fig. 15] Fig. 15 is a block diagram depicting the overall structure of a position detection system according to a third embodiment of the present invention.

[Fig. 16] Fig. 16 is a flowchart illustrating calibration by a position detection method using the position detection system in Fig. 15.

[Fig. 17] Fig. 17 is a flowchart illustrating the first half of actual measurement by the position detection method in Fig. 16.

[Fig. 18] Fig. 18 is a flowchart continued from the actual measurement in Fig. 17.

[Fig. 19] Fig. 19 is a flowchart continued from the actual measurement in Fig. 18.

[Fig. 20] Fig. 20 is a block diagram depicting the overall structure of a position detection system according to a fourth embodiment of the present invention.

[Fig. 21] Fig. 21 is a block diagram depicting the detailed structure of the position detection system in Fig. 20.

[Fig. 22] Fig. 22 is a flowchart illustrating waveform generation by a position detection method using the position detection system in Fig. 21.

[Fig. 23] Fig. 23 is a flowchart illustrating setting of read-out timing by the position detection method in Fig. 22.

[Fig. 24] Fig. 24 is a flowchart illustrating the first half of actual measurement by the position detection method using the position detection system in Fig. 22.

[Fig. 25] Fig. 25 is a flowchart continued from the actual measurement in Fig. 24.

[Fig. 26] Fig. 26 is a flowchart continued from the actual measurement in Fig. 25.

[Fig. 27] Fig. 27 is a structural diagram of a resonance circuit including a magnetic induction coil, for illustrating the setting of a position-calculating frequency in each embodiment.

Explanation of Reference Signs:

$f_0$: resonance frequency (first position-calculating frequency)

$f_1$, $f_2$: first position-calculating frequency

$f_3$, $f_4$: second position-calculating frequency

1, 40, 50, 60: position detection system

2: endoscope apparatus (endoscope)

2a: inserting section

3: capsule medical device (second marker)

3': second capsule medical device (capsule medical device, second marker)

4, 62: marker coil (first marker)

5: magnetic induction coil

6: magnetic-field detection section

24: frequency-selecting section (extracting section)

22: position/direction analyzing section

30: extraction/calculation section (extracting section)

41: magnetic-field generating device (magnetic-field generating unit)

61: first capsule medical device (capsule medical device)

71: three-axis Helmholtz coil unit (propulsion-magnetic-field generating unit)

72: Helmholtz-coil driver (propulsion-magnetic-field control section)

100: medical-device guidance system

150: permanent magnet (magnetic-field acting section)

Best Mode for Carrying Out the Invention

[First embodiment]

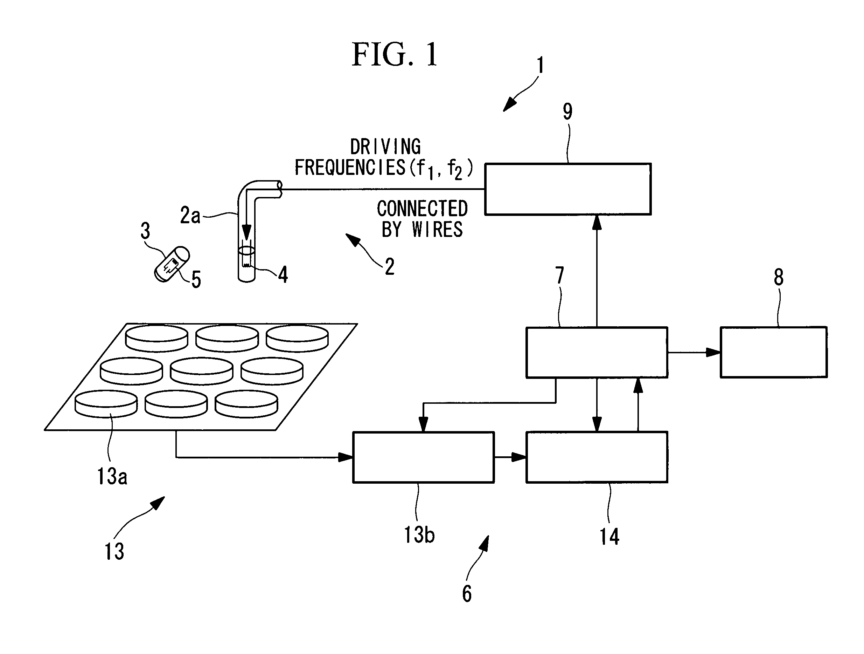

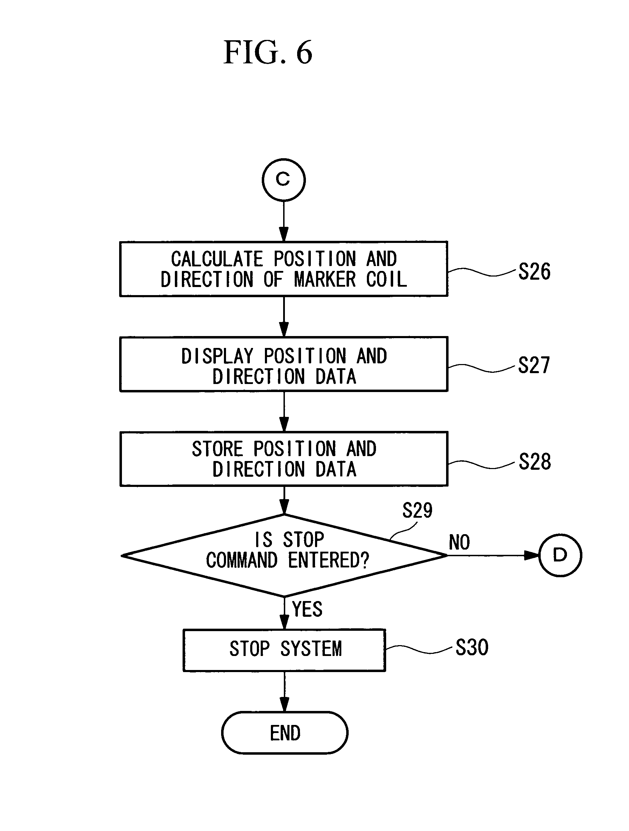

A position detection system 1 according to a first embodiment of the present invention will now be described with reference to Figs. 1 to 6.

The position detection system 1 according to this embodiment is a system that includes an endoscope apparatus 2, having an inserting section 2a inserted into a body cavity, and a capsule medical device 3 delivered into the body cavity. The position detection system 1 includes a marker coil (first marker) 4 disposed at a tip portion of the inserting section 2a of the endoscope apparatus 2, a magnetic induction coil (second marker) 5 disposed in the capsule medical device 3, a position detection apparatus 6 that detects the position of the marker coil 4, a control section 7 that controls these components, and a display device 8 that displays a result of detection by the position detection apparatus 6.

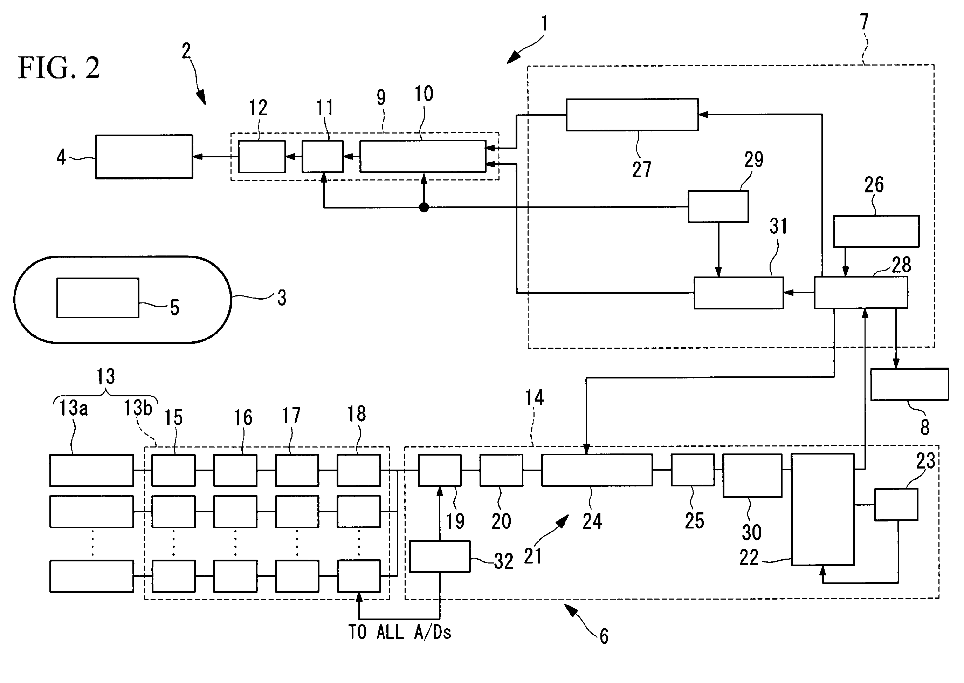

As shown in Fig. 2, the endoscope apparatus 2 is provided with a marker-driving circuit 9 that causes the marker coil 4 to produce a first alternating magnetic field in response to a command signal from the control section 7. The marker-driving circuit 9 includes a waveform data memory 10 that stores a magnetic-field waveform for the first alternating magnetic field to be produced by the marker coil 4, a D/A converter 11, and an amplifier 12.

The above-described marker coil 4 is driven by the marker-driving circuit 9 to produce a first alternating magnetic field having a single set of first position-calculating frequencies $f_1$ and $f_2$ that are substantially equal frequencies away from a resonance frequency $f_0$, which is input via an input device to be described later, with the first position-calculating frequencies $f_1$ and $f_2$ being on either side of the resonance frequency $f_0$.

The capsule medical device 3 is provided with a resonance circuit that includes the above-described magnetic induction coil 5 and that has the resonance frequency $f_0$, which is a substantially central frequency between the above-described single set of first position-calculating frequencies $f_1$ and $f_2$. The magnetic induction coil 5 produces an induced magnetic field in response to the first alternating magnetic field supplied from outside.

The above-described position detection apparatus 6 is disposed outside the body of a subject into which the endoscope apparatus 2 and the capsule medical device 3 are inserted. The position detection apparatus 6 includes a magnetic-field detection section 13 that detects magnetic fields produced from the marker coil 4 and the magnetic induction coil 5 and a position-calculating section 14 that calculates the positions and the directions of the endoscope apparatus 2 and the capsule medical device 3 based on the magnetic fields detected by the magnetic-field detection section 13.

The above-described magnetic-field detection section 13 includes a plurality of sense coils 13a and a receiving circuit 13b that receives an output signal from each of the sense coils 13a.

The sense coils 13a are each an air-core coil and are arranged in a square composed of one set of nine coils so as to face a working space for the tip of the inserting section 2a of the endoscope apparatus 2 and for the capsule medical device 3.

The receiving circuit 13b includes low-pass filters (LPFs) 15 that remove high-frequency components of AC voltages having information about the position of the endoscope apparatus 2, amplifiers (AMPs) 16 that amplify the AC voltages from which high-frequency components have been removed, band-pass filters (BPFs) 17 that transmit only predetermined frequency ranges of the amplified AC voltages, and A/D converters 18 that convert the AC voltages that have passed through the band-pass filters 17 into digital signals. As a result, the magnetic fields detected in the magnetic-field detection section 13 are output as magnetic-field signals composed of digital signals.

The above-described position-calculating section 14 includes a first memory 19 that stores the magnetic-field signals output from the receiving circuit 13b of the magnetic-field detection section 13, an FFT-processing circuit 20 that applies frequency analysis processing to the magnetic-field signals, an extracting section 21 that extracts predetermined magnetic-field information from a result of frequency analysis processing of the magnetic-field signals, a position/direction analyzing section 22 that calculates the positions and the directions of the endoscope apparatus 2 and the capsule medical device 3 based on the extracted magnetic-field information, and a second memory 23 that stores the calculated positions and directions of the endoscope apparatus 2 and the capsule medical device 3. In addition, the position-calculating section 14 is provided with a clock 32 that oscillates a clock signal for synchronizing all the A/D converters 18 in the above-described receiving circuit 13b with the position-calculating section 14.

The above-described extracting section 21 includes a frequency-selecting section 24 that receives from the control section 7 the first position-calculating frequencies $f_1$ and $f_2$, which are frequency components of a signal produced by the marker-driving circuit 9, and that extracts magnetic-field information having the first position-calculating frequencies $f_1$ and $f_2$ from among the magnetic-field information obtained by frequency analysis processing of the magnetic-field signals; a third memory 25 that stores a single set of magnetic-field information at the first position-calculating frequencies $f_1$ and $f_2$ extracted by the frequency-selecting section 24; and an extraction/calculation section 30 that extracts a signal from each of the sense coils 13a for calculating the positions of the marker coil 4 and the magnetic induction coil 5. The phrase "magnetic-field information at the first position-calculating frequencies $f_1$ and $f_2$" refers to the absolute values of the magnetic fields at the first position-calculating frequencies $f_1$ and $f_2$.

The above-described extraction/calculation section 30 calculates the sum and the difference between the intensity of the magnetic-field information at the first position-calculating frequency $f_1$ (first detection-magnetic-field component) and the intensity of the magnetic-field information at the first position-calculating frequency $f_2$ (first detection-magnetic-field component), i.e., the intensities of the magnetic-field information at the first position-calculating frequencies $f_1$ and $f_2$ that are stored in the third memory 25 and have been extracted by the above-described frequencyselection circuit.

The above-described position/direction analyzing section 22 calculates the position and the direction of the marker coil 4 of the endoscope apparatus 2 based on the sum of the intensities of the single set of magnetic-field information calculated in the above-described extraction/calculation section 30 and calculates the position and the direction of the magnetic induction coil 5 of the capsule medical device based on the difference between the intensities of the single set of magnetic-field information.

The above-described control section 7 includes an input device 26 used for various input operations; a waveform-data generator 27 that calculates a magnetic-field waveform to be produced from the marker coil 4 based on the resonance frequency of the magnetic induction coil 5 input via the input device 26; and a control circuit 28 that sets first position-calculating frequencies based on the input resonance frequency and transfers them to the waveform-data generator 27. Furthermore, the control section 7 further includes a clock 29 that produces a predetermined clock signal and a trigger generator 31 that produces a trigger signal based on the clock signal.

The control circuit 28 instructs the trigger generator 31 to produce a trigger signal for the marker-driving circuit 9. In addition, the above-described waveform-data generator 27 transfers the generated magnetic-field waveform to the waveform data memory 10 of the marker-driving circuit 9.

A method for detecting the positions of the tip of the endoscope apparatus 2 and the capsule medical device 3 using the position detection system 1 according to this embodiment with the above-described structure will be described below.

In order to detect the positions and the directions of the tip of the endoscope apparatus 2 and the capsule medical device 3 using the position detection system 1 according to this embodiment, the positions and the directions of the marker coil 4 at the tip of the endoscope apparatus 2 and of the magnetic induction coil 5 in the capsule medical device 3 are detected.

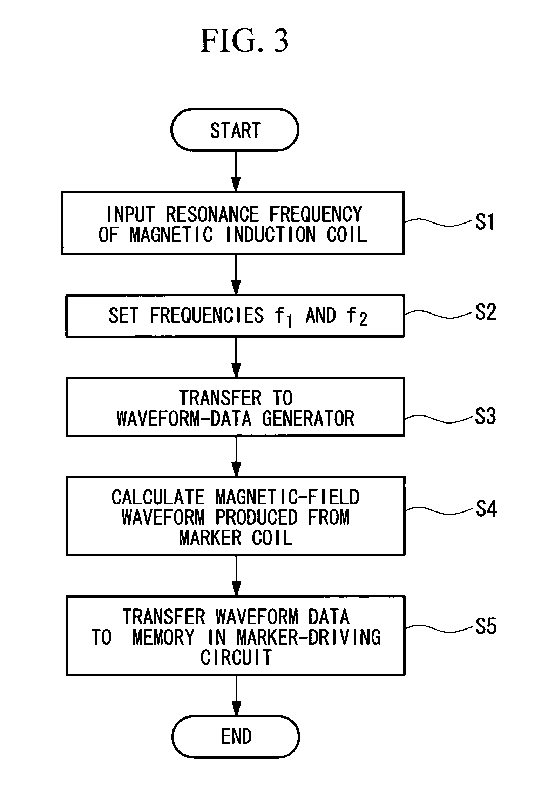

First, a magnetic-field waveform to be produced from the marker coil 4 is produced and stored in the waveform data memory 10 of the marker-driving circuit 9. The generation of a magnetic-field waveform starts according to the flow shown in Fig. 3. First, the resonance frequency $f_0$ of the magnetic induction coil 5 is input via the input device 26 (step S1). The control circuit 28 sets a single set of first position-calculating frequencies $f_1$ and $f_2$ that are away from the input resonance frequency $f_0$ by substantially equal frequencies, with the first position-calculating frequencies $f_1$ and $f_2$ being on either side of the resonance frequency $f_0$ (step S2). Then, the control circuit 28 transfers the set first position-calculating frequencies $f_1$ and $f_2$ to the waveform-data generator 27 (step S3). Doing so starts the generation of a magnetic-field waveform.

In the waveform-data generator 27, a magnetic-field waveform to be produced from the marker coil 4 based on the transferred single set of first position-calculating frequencies $f_1$ and $f_2$ is calculated using Expression (1) shown below (step S4). Thereafter, the calculated waveform data is transferred to the marker-driving circuit 9 and is then stored in the waveform data memory 10 (step S5).
$$B_{m1} = B_1 \times \sin(2\pi f_1 t) + B_2 \times \sin(2\pi f_2 t) \ldots (1)$$

where $B_1$ and $B_2$ are set in accordance with the characteristics of the sense coils 13a so that the magnetic-field components at the frequencies $f_1$ and $f_2$ exhibit the same level of signal intensity when detected by the sense coils 13a. ($B_1$ and $B_2$ are set so that $B_1 \times f_1 = B_2 \times f_2$ if the sense coils 13a are ideal coils. Alternatively, the frequency characteristics of the sense coils 13a may be pre-measured to set $B_1$ and $B_2$ in accordance with the pre-measured frequency characteristics.)

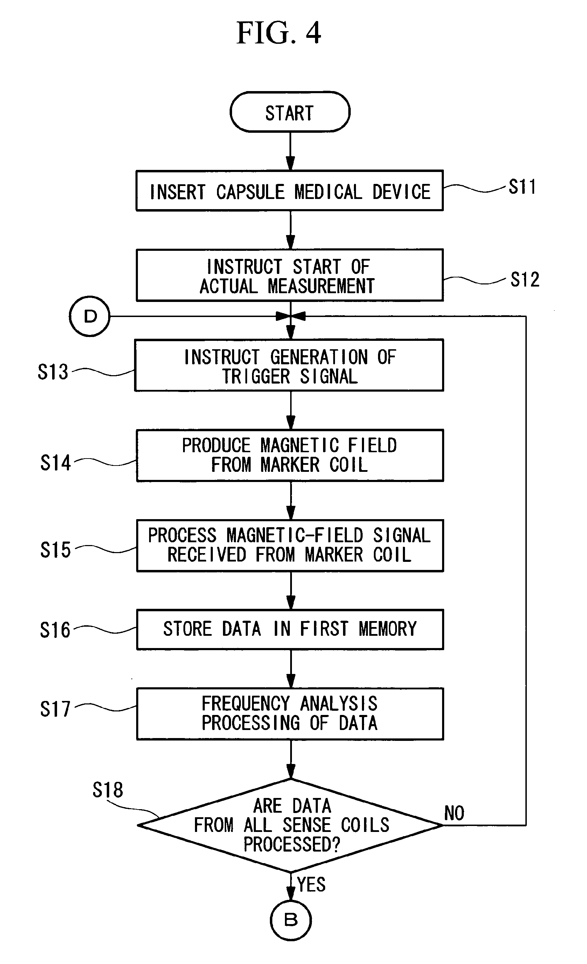

As shown in Figs. 4 to 6, actual measurement starts when a command for starting actual measurement is entered on the input device 26 (step S12) with the endoscope apparatus 2 and the capsule medical device 3 being disposed in the body cavity (step S11). The control circuit 28 instructs the trigger generator 31 to produce a trigger signal for the marker-driving circuit 9, and the trigger generator 31 produces a trigger signal (step S13).

The marker-driving circuit 9 sequentially generates magnetic-field-generation driving signals in synchronization with the clock signal based on the waveform data stored in the waveform data memory 10 and outputs them to the marker coil 4. The marker coil 4 produces the first alternating magnetic field based on the input magnetic-field-generation driving signals (step S14).

The receiving circuit 13b applies low-pass filtering with the low-pass filters 15, amplification with the amplifiers 16, and band-pass filtering with the band-pass filters 17 to the magnetic-field signals, associated with the first alternating magnetic field from the marker coil 4 and detected by the sense coils 13a, and then performs A/D conversion in synchronization with the clock signal from the clock 32 (step S15).

Each of the magnetic-field signals that have been subjected to A/D conversion is stored in the first memory 19 of the position-calculating section 14 (step S16). Then, it is determined whether or not a number of items of data required to perform frequency analysis processing are accumulated in the first memory 19, and if the required number of items of data are accumulated, the FFT-processing circuit 20 reads out magnetic-field signal data from the first memory 19 of the position-calculating section 14 and performs frequency analysis processing (step S17). Thereafter, it is determined whether or not this frequency analysis processing has been applied to the data from all the sense coils 13a (step S18), and if data from all the sense coils 13a have not been processed, steps S13 to S17 are repeated.

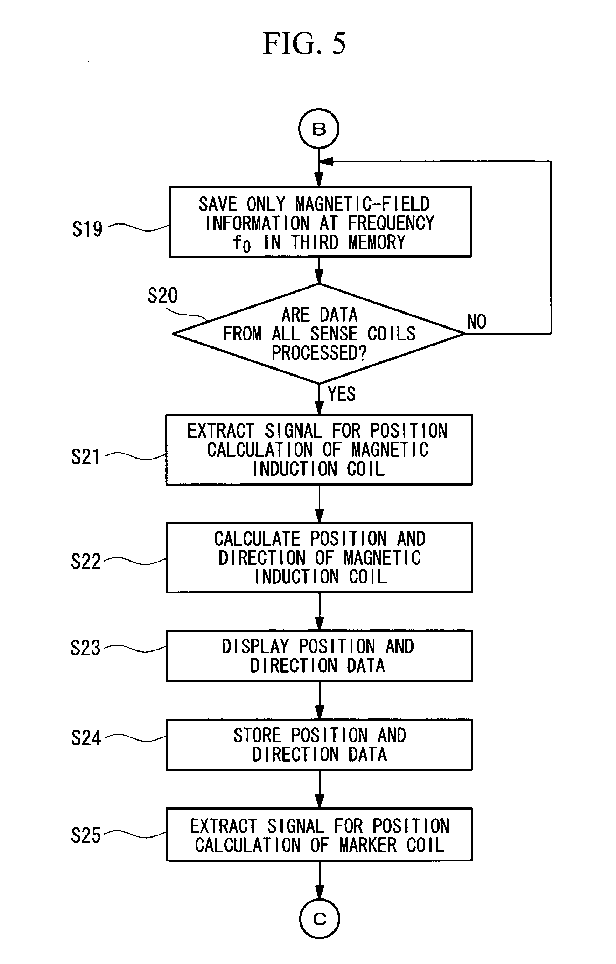

When the data from all the sense coils 13a have been subjected to frequency analysis processing, the frequency-selecting section 24 extracts, based on the result of processing, only the magnetic-field information at the first position-calculating frequencies $f_1$ and $f_2$ of the first alternating magnetic field produced from the marker coil 4 and stores it in the third memory 25 in association with the first position-calculating frequencies $f_1$ and $f_2$, as shown in Fig. 5 (step S19). This processing is applied to the magnetic-field signals from all the sense coils 13a (step S20).

In the extraction/calculation section 30, the signal from each of the sense coils 13a for calculating the position of the magnetic induction coil 5 is extracted based on the Expressions shown below (step S21).

$$V_{m2}{}^1 = V^{f1-1} - V^{f2-1}$$

$$V_{m2}{}^2 = V^{f1-2} - V^{f2-2}$$

$$\cdots$$

$$V_{m2}{}^N = V^{f1-N} - V^{f2-N}$$

In the above Expressions, $V^{f1-N}$ represents the absolute value of the magnetic-field intensity at the first position-calculating frequency f1 detected by the N-th sense coil 13a, and $V^{f2-N}$ indicates the absolute value of the magnetic-field intensity at the first position-calculating frequency $f_2$ detected by the N-th sense coil 13a. Furthermore, $V_{m2}{}^N$ represents a signal for performing position calculation of the magnetic induction coil 5 calculated based on the absolute values of the magnetic-field intensity detected by the N-th sense coil 13a.

In this case, the first terms of the Expressions for $V_{m2}{}^1$ through $V_{m2}{}^N$ correspond to magnetic-field information at the first position-calculating frequency $f_1$ (first detection-magnetic-field components). Here, the first term of the Expression for $V_{m2}{}^1$, that is, the signal detected by the first sense coil 13a at the frequency $f_1$, contains a signal with the frequency

$f_1$ of the first alternating magnetic field output from the marker coil 4, as well as a signal with the frequency $f_1$ of the induced magnetic field generated by the magnetic induction coil 5 in response to the first alternating magnetic field from the marker coil 4 (induced magnetic field associated with the first alternating magnetic field).

Furthermore, the second terms of the Expressions for $V_{m2}^1$ through $V_{m2}^N$ correspond to magnetic-field information at the first position-calculating frequency $f_2$ (first detection-magnetic-field components). Here, the second term of the Expression for $V_{m2}^1$, that is, the signal detected by the second sense coil 13a at the frequency $f_2$, contains a signal with the frequency $f_2$ of the first alternating magnetic field output from the marker coil 4, as well as a signal with the frequency $f_2$ of the induced magnetic field generated by the magnetic induction coil 5 in response to the first alternating magnetic field from the marker coil 4 (induced magnetic field associated with the first alternating magnetic field).

Here, because the resonance frequency $f_0$ of the magnetic induction coil 5 is a substantially central frequency between the single set of the position-calculating frequencies $f_1$ and $f_2$, the signals with the frequencies $f_1$ and $f_2$ of the induced magnetic field associated with the first alternating magnetic field have the characteristic that they differ from each other in the magnitude relationship of intensity with respect to the first alternating magnetic field and that they have substantially the same absolute value of the intensity. On the other hand, the signals with the frequencies $f_1$ and $f_2$ of the first alternating magnetic field are set so as to exhibit the same level of signal intensity when the magnetic-field components at the frequencies $f_1$ and $f_2$ are detected by the sense coils 13a, as described above, in step S4 serving as the process of generating a magnetic-field waveform. Because of this, when the difference between the first term and the second term of each of the Expressions for $V_{m2}^1$ through $V_{m2}^N$, that is, the difference between the single set of first detection-magnetic-field components, is calculated, the signals of the first alternating magnetic field are cancelled out, whereas the signals of the induced magnetic field associated with the first alternating magnetic field remain, without being cancelled out.

In this manner, the signals of the first alternating magnetic field can be cancelled out by calculating the difference between the absolute values of the magnetic-field intensity at the single set of first position-calculating frequencies $f_1$ and $f_2$, which are substantially the same frequency away from the resonance frequency $f_0$, with the first position-calculating frequencies $f_1$ and $f_2$ being on either side of the resonance frequency $f_0$. As a result, the signals of the induced magnetic field produced by the first alternating magnetic field can be extracted easily (step S21).

The position/direction analyzing section 22 calculates the position and the direction of the magnetic induction coil 5 from $V_{m2}^1$, $V_{m2}^2$, ... $V_{m2}^N$ obtained in the extraction/calculation section 30 (step S22).

Data on the calculated position and direction of the magnetic induction coil 5 is sent to the control circuit 28 and displayed on the display device 8 (step S23). Thereafter, the data on the calculated position and direction is accumulated in the second memory 23 (step S24).

Next, in the extraction/calculation section 30, the signal from each of the sense coils 13a for calculating the position of the marker coil 4 is calculated based on the Expressions shown below (step S25).

$$V_{m1}^1 \ = \ V^{f1-1} \ + \ V^{f2-1},$$

$$V_{m1}^2 \ = \ V^{f1-2} \ + \ V^{f2-2},$$

$$. \ . \ .$$

$$V_{m1}^N \ = \ V^{f1-N} \ + \ V^{f2-N}$$

In this case, the first terms of the Expressions for $V_{m1}^1$ through $V_{m1}^N$ correspond to magnetic-field information at the first position-calculating frequency $f_1$ (first detection-magnetic-field components). Here, the first term of the Expression for $V_{m1}^1$, that is, the signal detected by the first sense coil 13a at the frequency $f_1$, contains a signal with the frequency $f_1$ of the first alternating magnetic field output from the marker coil 4, as well as a signal with the frequency $f_1$ of the induced magnetic field generated by the magnetic induction coil 5 in response to the first alternating magnetic field from the marker coil 4 (induced magnetic field associated with the first alternating magnetic field).

Furthermore, the second terms of the Expressions for $V_{m1}^1$ through $V_{m1}^N$ correspond to magnetic-field information at the first position-calculating frequency $f_2$ (first detection-magnetic-field components). Here, the second term of the Expression for $V_{m1}^1$, that is, the signal detected by the second sense coil 13a at the frequency $f_2$, contains a signal with the frequency $f_2$ of the first alternating magnetic field output from the marker coil 4, as well as a signal with the frequency $f_2$ of the induced magnetic field generated by the magnetic induction coil 5 in response to the first alternating magnetic field from the marker coil 4 (induced magnetic field associated with the first alternating magnetic field).

Here, the signals with the frequencies $f_1$ and $f_2$ of the induced magnetic field associated with the first alternating magnetic field have the characteristic that they differ from each other in the magnitude relationship of intensity with respect to the first alternating magnetic field and that they have substantially the same absolute value of the intensity. Because of this, when the sum of the first term and the second term of each of the Expressions for $V_{m1}^1$ through $V_{m1}^N$, that is, the sum of the single set of first detection-magnetic-field components, is calculated, the signals of the induced magnetic field associated with the first alternating magnetic field are cancelled out, whereas the signals of the first alternating magnetic field remain, without being cancelled out.
In this manner, the signals of the induced magnetic field associated with the first alternating magnetic field can be cancelled out by adding the absolute values of the magnetic-field intensity at the single set of first position-calculating frequencies $f_1$ and $f_2$, which are substantially the same frequency away from the resonance frequency $f_0$, with the first position-calculating frequencies $f_1$ and $f_2$ being on either side of the resonance frequency $f_0$. As a result, the signals of the first alternating magnetic field can be extracted easily.

The position/direction analyzing section 22 calculates the position and the direction of the marker coil 4 from $V_{m1}^1$, $V_{m1}^2$, ... $V_{m1}^N$ obtained in the extraction/calculation section 30 (step S26).
Data on the calculated position and direction of the marker coil 4 is sent to the control circuit 28 and displayed on the display device 8 (step S27). Thereafter, the data on the calculated position and direction is accumulated in the second memory 23 (step S28).

Then, it is checked whether or not a command for terminating position detection has been input on the input device 26 (step S29), and if a command has been input, generation of a trigger signal from the trigger generator 31 is terminated to stop the operation of the position detection system 1 (step S30). On the other hand, if no termination command has been input, the flow returns to step S13 to continue position detection.

In this case, for the initial values for iterated arithmetic operations of the positions and directions of the magnetic induction coil 5 and the marker coil 4, the calculation results of the positions and the directions of the magnetic induction coil 5 and the marker coil 4 that have previously been calculated and stored in the second memory 23 are used. By doing so, the convergence time of iterated arithmetic operations can be reduced to calculate the positions and the directions in a shorter period of time.

In this manner, according to the position detection system 1 of this embodiment and a position detection method using the system 1, the signal from the marker coil 4 and the signal from the magnetic induction coil 5 can be completely separated from each other based on position information of both the signals. Consequently, the positions and directions of the marker coil 4 and the magnetic induction coil 5, namely, the positions and directions of the tip of the inserting section 2a of the endoscope apparatus 2 and the capsule medical device 3 disposed in the body cavity, can be obtained precisely.

[Second embodiment]

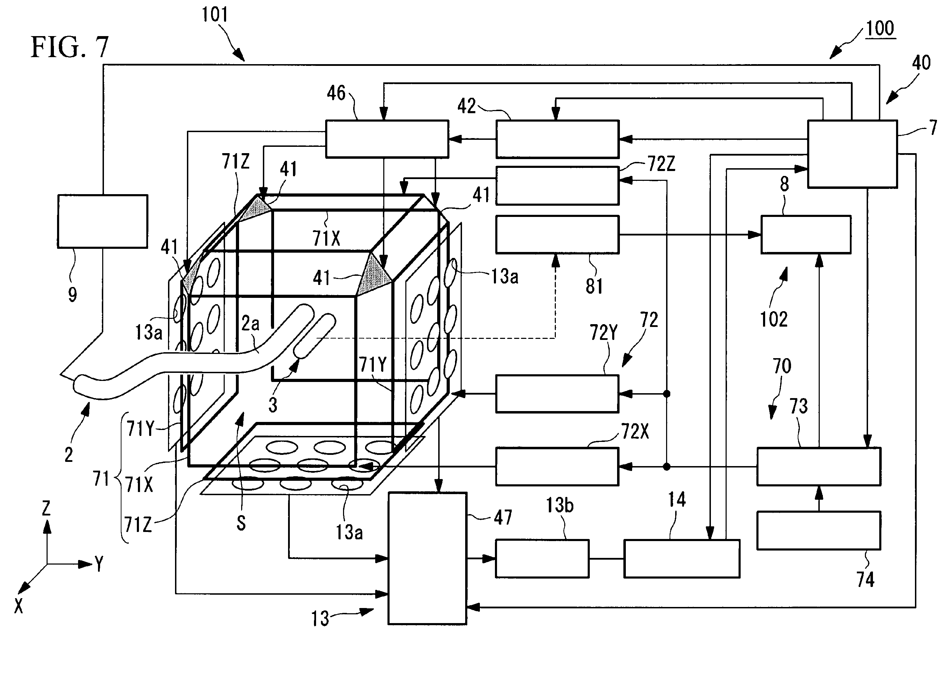

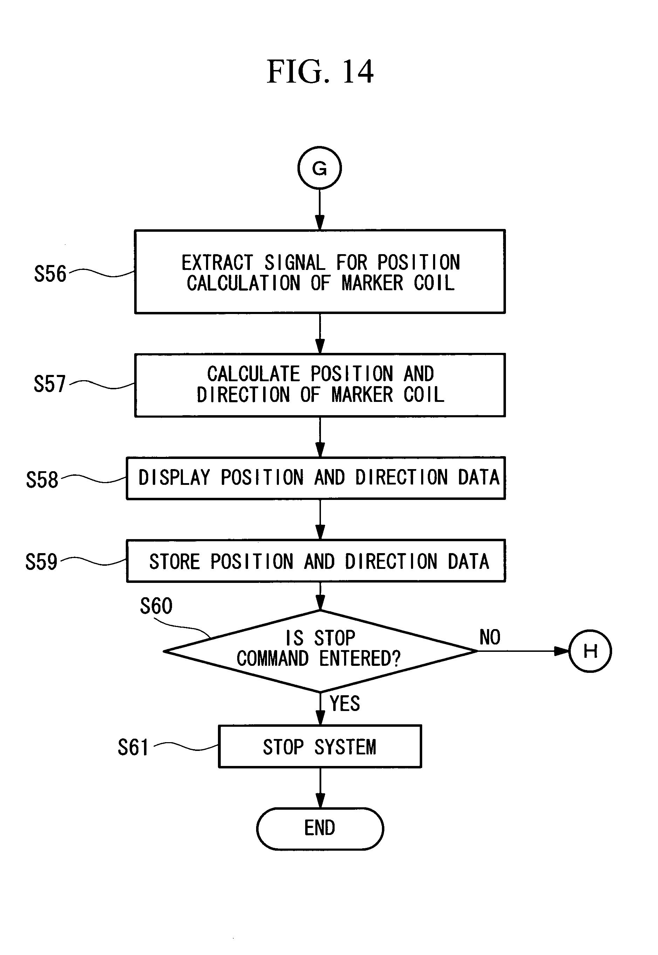

A position detection system 40 according to a second embodiment of the present invention will now be described with reference to Figs. 7 to 14.
In the description of this embodiment, the same components as those of the position detection system 1 according to the first embodiment are denoted by the same reference numerals, and thus an explanation thereof will be omitted.

As shown in Fig. 7, the position detection system 40 according to this embodiment is provided in a medical-device guidance system 100. The medical-device guidance system 100 includes the endoscope apparatus 2 and the capsule medical device 3 that are introduced, per oral or per anus, into the body cavity of a subject; the position detection system 40; a magnetic induction apparatus 101 that guides the capsule medical device 3 based on the detected position and direction and an operator support; and an image display device 102 that displays an image signal transmitted from the capsule medical device 3.

As shown in Fig. 7, the magnetic induction apparatus 101 includes a three-axis Helmholtz coil unit (propulsion-magnetic-field generating unit) 71 that produces parallel external magnetic fields (rotating magnetic fields) for driving the capsule medical device 3; a Helmholtz-coil driver 72 that amplifies and controls an electrical current to be supplied to the three-axis Helmholtz coil unit 71; a magnetic field control circuit (propulsion-magnetic-field control section) 73 that controls the direction of an external magnetic field for driving the capsule medical device 3; and an input device 74 that outputs to the magnetic field control circuit 73 the direction of movement of the capsule medical device 3 input by the operator.

Although the term "three-axis Helmholtz coil unit 71" is used in this embodiment, it is not necessary that Helmholtz-coil conditions be strictly satisfied. For example, the coils need not be circular but may be substantially rectangular, as shown in Fig. 7. Furthermore, the gaps between opposing coils do not need to satisfy Helmholtz-coil conditions, as long as the function of this embodiment is achieved.

As shown in Fig. 7, the three-axis Helmholtz coil unit 71 is formed in a substantially rectangular shape. In addition, the three-axis Helmholtz coil unit 71 includes three-pairs of mutually opposing Helmholtz coils (electromagnets) 71X, 71Y, and 71Z, and each pair of Helmholtz coils 71X, 71Y, and 71Z is disposed so as to be substantially orthogonal to the X, Y, and Z axes in Fig. 7. The Helmholtz coils disposed substantially orthogonally with respect to the X, Y, and Z axes are denoted as the Helmholtz coils 71X, 71Y, and 71Z, respectively.

Furthermore, the Helmholtz coils 71X, 71Y, and 71Z are disposed so as to form a substantially rectangular space S in the interior thereof. As shown in Fig. 7, the space S serves as a working space (also referred to as the working space S) of the capsule medical device 3 and is the space in which the subject is placed.

The Helmholtz-coil driver 72 includes Helmholtz-coil drivers 72X, 72Y, and 72Z for controlling the Helmholtz coils 71X, 71Y, and 71Z, respectively.

The magnetic field control circuit 73 receives from the position detection system 40 (described later) data representing the current direction of the capsule medical device 3 (the direction along the longitudinal axis R of the capsule medical device 3), as well as a direction-of-movement command for the capsule medical device 3 input by the operator on the input device 74. Then, from the magnetic field control circuit 73, signals for controlling the Helmholtz-coil drivers 72X, 72Y, and 72Z are output, rotational phase data of the capsule medical device 3 is output to the display device 8, and electrical current data to be supplied to each of the Helmholtz-coil drivers 72X, 72Y, and 72Z is output.

Furthermore, for example, a joystick (not shown in the figure) is provided as the input device 74, so that the movement direction of the capsule medical device 3 can be specified by tilting the joystick.

As mentioned above, for the input device 74, a joystick-type device may be used, or another type of input device may be used, such as an input device that specifies the direction of movement by pushing direction-of-movement buttons.

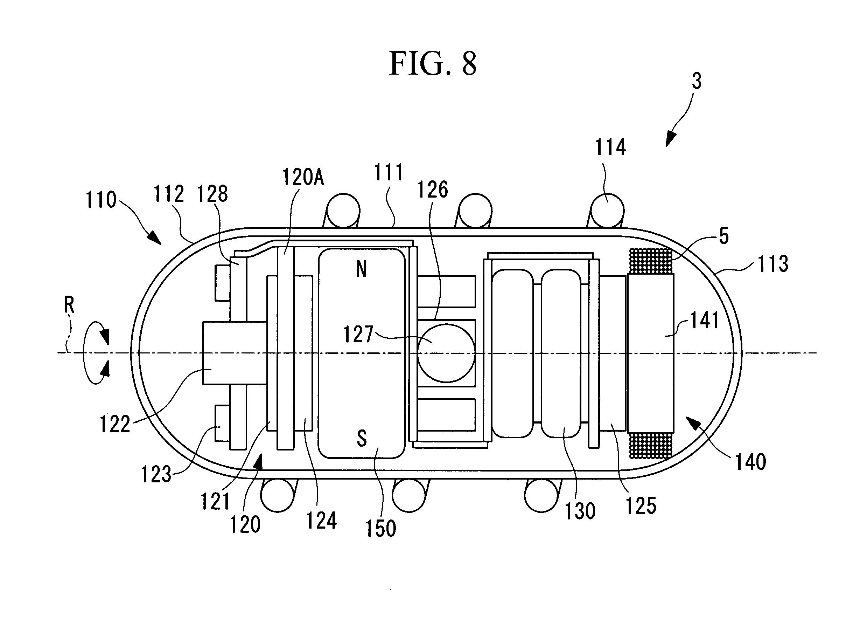

As shown in Fig. 8, the capsule medical device 3 includes an enclosure 110 accommodating various types of devices therein; an imaging section 120 that acquires an image of the internal surface of a body cavity tract of the subject; a battery 130 that powers the imaging section 120; an induced-magnetic-field generating unit 140 that produces an alternating magnetic field with a magnetic-field generating device 41 (described later); and a permanent magnet (magnetic-field acting section) 150 that drives the capsule medical device 3 in response to the external magnetic field produced by a magnetic induction apparatus 70.

The enclosure 110 includes an infrared-transmitting cylindrical capsule main body (hereinafter, referred to simply as the "main body") 111 whose central axis is defined by the longitudinal axis R of the capsule medical device 3; a transparent, hemispherical front end portion 112 covering the front end of the main body 111; and a hemispherical rear end portion 113 covering the rear end of the main body, to form a sealed capsule container with a watertight construction.

Furthermore, a helical part 114 made of a wire having a circular cross-section is helically wound about the longitudinal axis R over the outer circumferential surface of the main body 111 of the enclosure 110.

When the permanent magnet 150 is rotated in response to the rotating external magnetic field produced by the magnetic induction apparatus 70, the helical part 114 is rotated about the longitudinal axis R along with the main body 111. As a result, the rotational motion about the longitudinal axis R of the main body 111 is transformed into a linear motion in the direction along the longitudinal axis R by means of the helical part 114, thereby making it possible to guide the capsule medical device 3 in the direction along the longitudinal axis R in the body passage.

The imaging section 120 includes a board 120A disposed substantially orthogonal to the longitudinal axis R; an image sensor 121 disposed on the surface of the board 120A at the front end portion 112 side; a lens group 122 that forms an image of an internal surface of a passage in the body cavity of the subject at the image sensor 121; an LED (light emitting diode) 123 that emits light onto the internal surface of the passage in the body cavity; a signal processing unit 124 disposed on the surface of the board 120A at the rear end portion 113 side; and a radio device 125 that transmits an image signal to the image display device 102.

The signal processing unit 124 is electrically connected to the battery 130 and is electrically connected to the image sensor 121 and the LED 123. Also, the signal processing unit 124 compresses the image signal acquired by the image sensor 121, temporarily stores it (memory), and transmits the compressed image signal to the exterior from the radio device 125, and in addition, it controls the on/off state of the image sensor 121 and the LED 123 based on signals from a switch unit 126 to be described later.

The image sensor 121 converts the image formed via the front end portion 112 and the lens group 122 into an electrical signal (image signal) and outputs it to the signal processing unit 124. A CMOS (Complementary Metal Oxide Semiconductor) device or a CCD, for example, can be used as this image sensor 121.

Moreover, a plurality of the LEDs 123 are disposed on a support member 128 positioned towards the front end portion 112 from the board 120A such that gaps are provided therebetween in the circumferential direction around the longitudinal axis R.

The image display device 102 includes an image receiving circuit 81 that receives image data sent from the capsule medical device 3 and the display device 8 that displays the received image data.

The permanent magnet 150 is disposed towards the rear end portion 113 from the signal processing unit 124. The permanent magnet 150 is disposed or polarized so as to have a magnetization direction (magnetic pole) in a direction orthogonal to the longitudinal axis R.

The switch unit 126 is disposed at the side of the permanent magnet 150 at the rear end portion 113 side. The switch unit 126 includes an infrared sensor 127 and is electrically connected to the signal processing unit 124 and the battery 130.

Also, a plurality of the switch units 126 are disposed in the circumferential direction about the longitudinal axis R at regular intervals, and the infrared sensor 127 is disposed so as to face the outside in the diameter direction. In this embodiment, an example has been described in which four switch units 126 are disposed, but the number of switch units 126 is not limited to four; any number may be provided.

The induced-magnetic-field generating unit 140, which is disposed at the side of the radio device 125 at the rear end portion 113 side, is composed of a core member (magnetic core) 141 made of ferrite formed in the shape of a cylinder whose central axis is substantially aligned with the longitudinal axis R, the magnetic induction coil 5 disposed at the outer circumferential part of the core member 141, and a capacitor (not shown in the figure) that is electrically connected to the magnetic induction coil 5 and that constitutes the resonance circuit.

In addition to ferrite, magnetic materials are suitable for the core member 141; iron, nickel, permalloy, cobalt or the like may be used for the core member. Furthermore, the magnetic induction coil 5 may be formed of an air-core coil without a magnetic core.

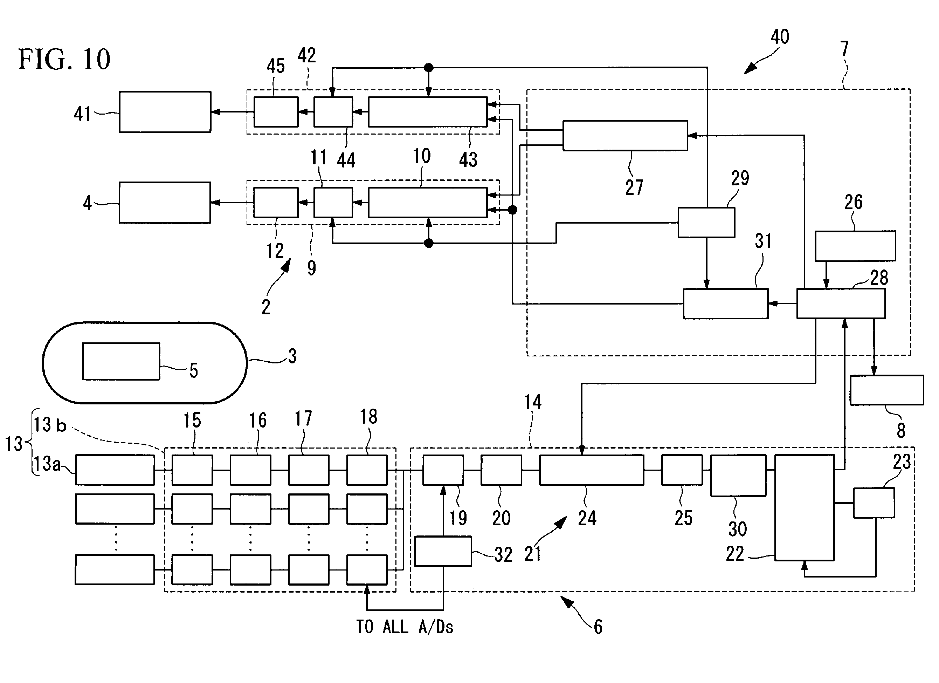

As shown in Figs. 7 and 10, the position detection system 40 according to this embodiment differs from the position detection system 1 according to the above-described first embodiment in that the position detection system 40 includes the magnetic-field generating device 41 that is disposed outside a working region of the magnetic induction coil 5 and that produces a second alternating magnetic field having the same frequency and phase as those of the above-described first alternating magnetic field, as well as a magnetic-field-generating-device driving circuit 42. The system 40 also differs from the system 1 in arithmetic operations performed in the position/direction analyzing section 22. In Fig. 10, reference numeral 43 denotes a waveform data memory, reference numeral 44 denotes a D/A converter, and reference numeral 45 denotes an amplifier. Furthermore, in Fig. 7, reference numeral 46 denotes a selector that selects the magnetic-field generating device 41, and reference numeral 47 denotes a sense-coil selector that selects the sense coils 13a.

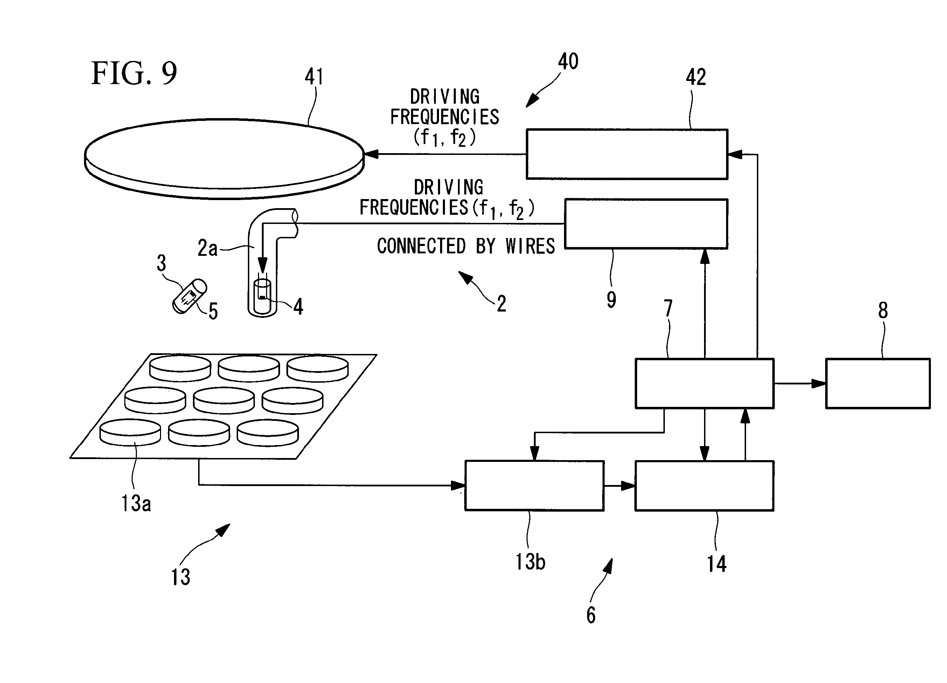

Figs. 9 and 10 depict a simplified form of the position detection system 40 according to this embodiment.

In order to detect the positions and the directions of the marker coil 4 at the tip of the endoscope apparatus 2 and the magnetic induction coil 5 in the capsule medical device 3 by using the position detection system 40 according to this embodiment, waveform data of the produced first and second alternating magnetic fields is generated and is stored in the waveform data memories 10 and 43, and then calibration is carried out with the capsule medical device 3 being disposed outside the working region.

Because not only is the first alternating magnetic field produced from the marker coil 4 but also the second alternating magnetic field is produced from the magnetic-field generating device 41, items of data on the generated magnetic field waveform are transferred to the waveform data memory 10 of the marker-driving circuit 9 and the waveform data memory 43 of the magnetic-field-generating-device driving circuit 42, respectively.

In the waveform-data generator 27, a magnetic-field waveform to be produced from the marker coil 4 based on the transferred single set of first position-calculating frequencies $f_1$ and $f_2$ is calculated using Expression (1) shown below.

$$B_{m1} = B_1 \times \sin(2\pi f_1 t) + B_2 \times \sin(2\pi f_2 t) \ \dots \ (1)$$

Also in the waveform-data generator 27, a magnetic-field waveform to be produced from the magnetic-field generating device 41 based on the transferred single set of first position-calculating frequencies $f_1$ and $f_2$ is calculated using Expression (2) below.

$$B_G = B_3 \times \sin(2\pi f_1 t) + B_4 \times \sin(2\pi f_2 t) \ \dots \ (2)$$

Thereafter, data on the calculated magnetic-field waveform $B_{m1}$ is transferred to the marker-driving circuit 9 and is then stored in the waveform data memory 10. Furthermore, data on the calculated magnetic-field waveform $B_G$ is transferred to the magnetic-field-generating-device driving circuit 42 and is then stored in the waveform data memory 43. The first and second alternating magnetic fields to be produced from the marker coil 4 and the magnetic-field generating device 41 correspond to the single set of first position-calculating frequencies $f_1$ and $f_2$, which are substantially the same frequency away from the resonance frequency $f_0$ of the magnetic induction coil 5, with the first position-calculating frequencies $f_1$ and $f_2$ being on either side of the resonance frequency $f_0$, and have the same phase.

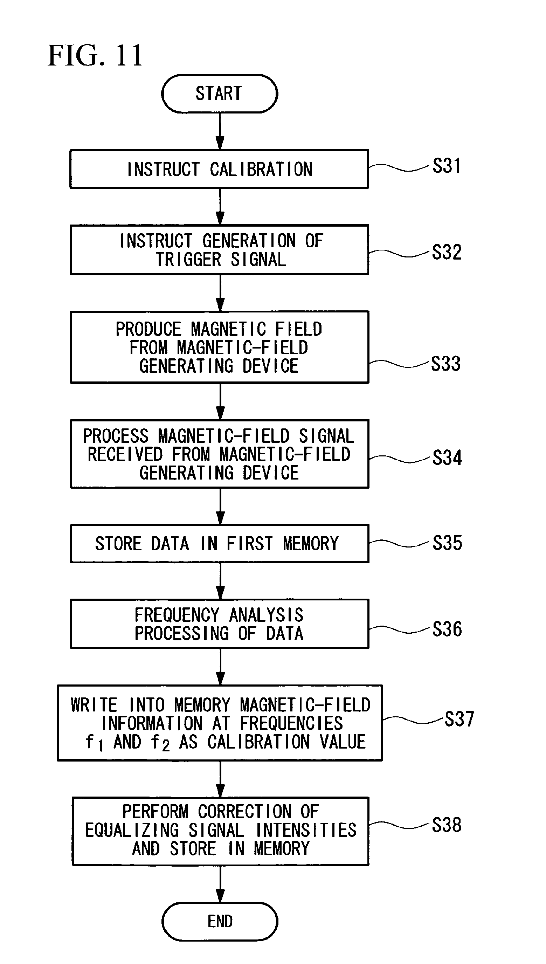

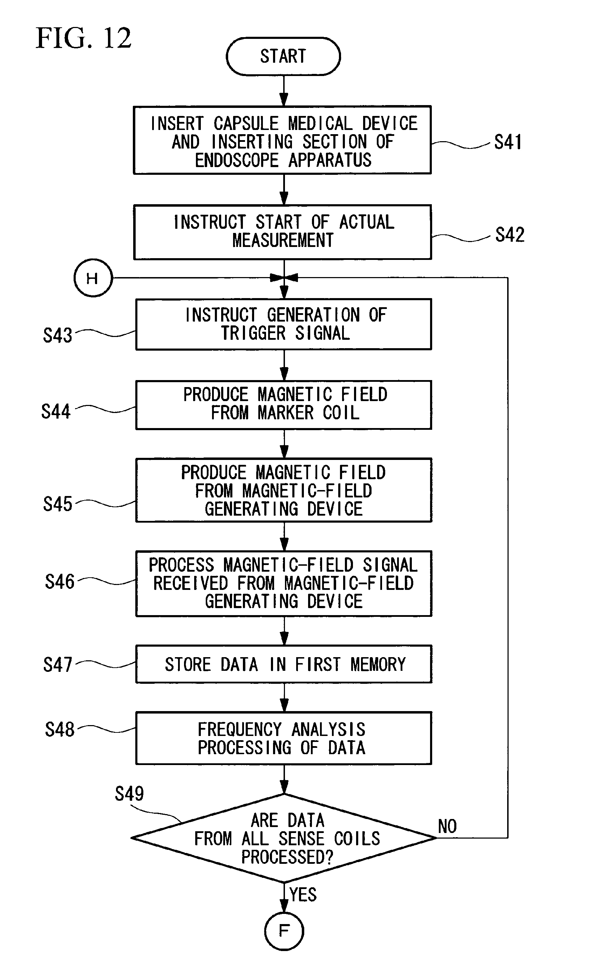

As shown in Figs. 11 and 12, calibration starts when a calibration command is input via the input device 26 while the tip of the inserting section 2a of the endoscope apparatus 2 is disposed in the body cavity and the capsule medical device 3 is not disposed in the body cavity (step S31). The control circuit 28 instructs the trigger generator 31 to produce a trigger signal for the magnetic-field-generating-device driving circuit 42. By doing so, a trigger signal is issued from the trigger generator 31 (step S32).

Based on the waveform data stored in the waveform data memory 43, the magnetic-field-generating-device driving circuit 42 that has received the trigger signal sequentially generates magnetic-field-generation driving signals in synchronization with the clock signal from the clock 29 and outputs them to the magnetic-field generating device 41. The magnetic-field generating device 41 produces the second alternating magnetic field based on the input magnetic-field-generation driving signals (step S33).

The receiving circuit 13b receives a magnetic-field signal associated with the second alternating magnetic field from the magnetic-field generating device 41 detected by each of the sense coils 13a; performs low-pass filtering, amplification, and band-pass filtering; and then performs A/D conversion in synchronization with.the clock signal from the clock 32 (step S34).

The magnetic-field signal that has been subjected to A/D conversion is stored in the first memory 19 of the position-calculating section 14 (step S35). Thereafter, it is determined whether or not a number of items of data required to perform frequency analysis processing are accumulated in the first memory 19, and if the required number of items of data are accumulated, frequency analysis processing is performed by the FFT-processing circuit 20 (step S36).

Based on the result of frequency analysis processing, the frequency-selecting section 24 extracts only the magnetic-field information at the first position-calculating frequencies $f_1$ and $f_2$ of the first alternating magnetic field produced from the marker coil 4 and the second alternating magnetic field produced from the magnetic-field generating device 41 and stores it in the third memory 25 in association with the frequencies $f_1$ and $f_2$ (step S37).

Let the signal intensities of the stored magnetic-field information at the first position-calculating frequencies $f_1$ and $f_2$ at this time be respectively represented as $V_0^{f1-1}$, $V_0^{f1-2}$, ... $V_0^{f1-N}$, $V_0^{f2-1}$, $V_0^{f2-2}$, ... $V_0^{f2-N}$, where superscripts $f_1$ and $f_2$ indicate frequency components and the subsequent superscripts 1, 2, ..., N indicate the numbers of the sense coils 13a. Furthermore, the term "magnetic-field information" means the absolute value of the result of FFT processing. The magnetic-field information at these first position-calculating frequencies $f_1$ and $f_2$ is stored in the third memory 25 as calibration values.

Here, the signal intensities at the frequency $f_1$ and the signal intensities at the frequency $f_2$ detected by all the sense coils 13a are corrected.
More specifically, the sum $\Sigma(V_0^{f1-N})$ of the signal components at the frequency $f_1$ detected by all the sense coils 13a and the sum $\Sigma(V_0^{f2-N})$ of the signal components at the frequency $f_2$ detected by all the sense coils 13a are obtained first. Then, the ratio of the sums of the signal components $\Sigma(V_0^{f1-N})/\Sigma(V_0^{f2-N})$ is obtained as a correction factor.

Subsequently, $V_0^{f2-1}$, $V_0^{f2-2}$, ... , $V_0^{f2-N}$ are replaced as shown below using the obtained correction factor by overwriting the third memory 25.

$$V_0 f^{2-1} \text{ is replaced by } V_0 f^{2-1} \times \Sigma(V_0{}^{f1-N})/\Sigma(V_0{}^{f2-N}).$$

$$V_0{}^{f2-2} \text{ is replaced by } V_0{}^{f2-2} \times \Sigma(V_0{}^{f1-N})/\Sigma(V_0{}^{f2-N}).$$

$$...$$

$$V_0{}^{f2-N} \text{ is replaced by } V_0{}^{f2-N} \times \Sigma(V_0{}^{f1-N})/\Sigma(V_0{}^{f2-N}).$$

Furthermore, the correction factor $\Sigma(V_0^{f1-N})/\Sigma(V_0^{f2-N})$ is also stored in the third memory 25 (step S38).

By doing so, $V_0^{f1-1}$ and $V_0^{f2-1}$ ($V_0^{f2-1} \times (V_0^{f1-N})/\Sigma(V_0^{f2-N})$ as a result of replacement) stored in the third memory 25 have substantially the same values. In other words, an operation is carried out for making the gain for the signal at the frequency $f_1$ from each of the sense coils 13a substantially the same as the gain for the signal at the frequency $f_2$.

Next, actual measurement starts when a command for starting actual measurement is entered on the input device 26 (step S42) with the endoscope apparatus 2 and the capsule medical device 3 being disposed in the body cavity (step S41), as shown in Figs. 12 to 14.
The control circuit 28 instructs the trigger generator 31 to produce a trigger signal for the marker-driving circuit 9 and the magnetic-field-generating-device driving circuit 42, and the trigger generator 31 produces a trigger signal (step S43).

The marker-driving circuit 9 sequentially generates magnetic-field-generation driving signals in synchronization with the clock signal based on the waveform data stored in the waveform data memory 10 and outputs them to the marker coil 4. The marker coil 4 produces the first alternating magnetic field based on the input magnetic-field-generation driving signals (step S44).

Furthermore, based on the waveform data stored in the waveform data memory 43, the magnetic-field-generating-device driving circuit 42 sequentially generates magnetic-field-generation driving signals in synchronization with the clock signal and outputs them to the magnetic-field generating device 41. The magnetic-field generating device 41 produces the second alternating magnetic field based on the input magnetic-field-generation driving signals (step S45).

The receiving circuit 13b applies low-pass filtering, amplification, and band-pass filtering to a magnetic-field signal associated with the first alternating magnetic field from the marker coil 4 and to a magnetic-field signal associated with the second alternating magnetic field from the magnetic-field generating device 41, i.e., the magnetic-field signals detected by each of the sense coils 13a, and then performs A/D conversion in synchronization with the clock signal from the clock 32 (step S46).

The magnetic-field signals that have been subjected to A/D conversion are stored in the first memory 19 of the position-calculating section 14 (step S47).

Then, it is determined whether or not a number of items of data required to perform frequency analysis processing are accumulated in the first memory 19, and if the required number of items of data are accumulated, the FFT-processing circuit 20 reads out signal data from the first memory 19 and carries out frequency analysis processing (step S48). Thereafter, it is determined whether or not the data from all the sense coils 13a have been subjected to this frequency analysis processing (step S49). If data from all sense coils 13a have not been processed, steps S43 to S48 are repeated.

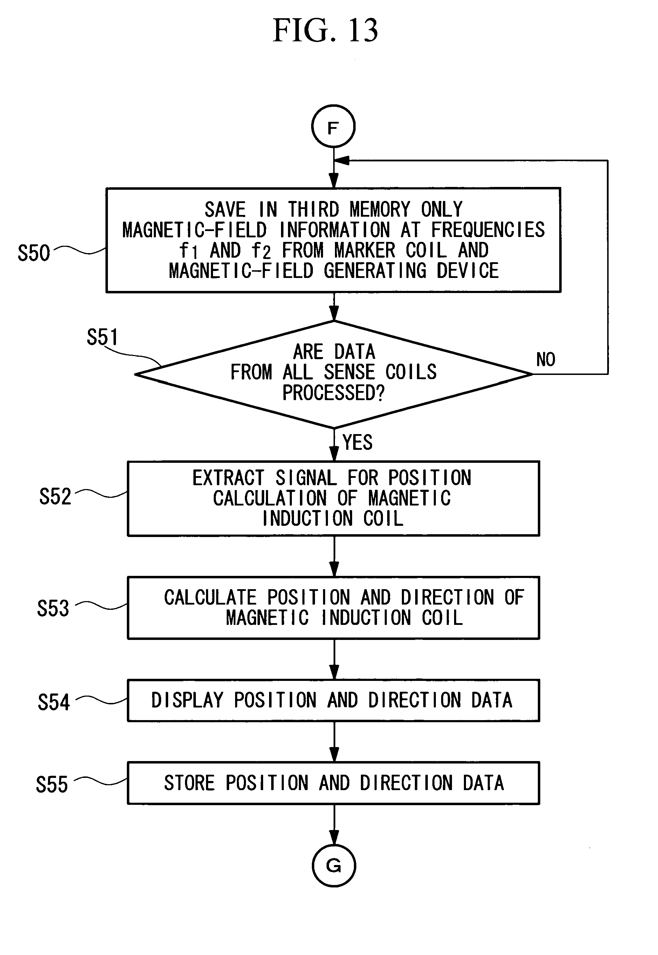

When the data from all the sense coils 13a have been subjected to frequency analysis processing, the frequency-selecting section 24 extracts, based on the result of processing, only the magnetic-field information at the first position-calculating frequencies $f_1$ and $f_2$ of the first alternating magnetic field produced from the marker coil 4 and the second alternating magnetic field produced from the magnetic-field generating device 41, as shown in Fig. 13, and stores it in the third memory 25 in association with the frequencies $f_1$ and $f_2$ (step S50). This processing is applied to the magnetic-field signals from all the sense coils 13a (step S51).

In the extraction/calculation section 30, the signal from each of the sense coils 13a for calculating the position of the magnetic induction coil 5 is extracted from the Expressions shown below (step S52).

$$V_{m2}^{1} = (V^{f1-1} - V_0^{f1-1}) - (V^{f2-1} \times \Sigma(V_0^{f1-N}) / \Sigma(V_0^{f2-N}) - V_0 f^{2-1})$$

$$V_{m2}^{2} = (V^{f1-2} - V_0^{f1-2}) - (V^{f2-2} \times \Sigma(V_0^{f1-N}) / \Sigma(V_0^{f2-N}) - V_0^{f2-2})$$

$$\cdots$$

$$V_{m2}^{N} = (V^{f1-N} - V_0^{f1-N}) - (V^{f2-N} \times \Sigma(V_0^{f1-N}) / \Sigma(V_0^{f2-N}) - V_0^{f2-N})$$

In this case, the first terms of the Expressions for $V_{m2}^{1}$ through $V_{m2}^{N}$ correspond to magnetic-field information at the first position-calculating frequency $f_1$ (first detection-magnetic-field components). Here, of the first term $(V^{f1-1} - V_0^{f1-1})$ of the Expression for $V_{m2}^{1}$, $V^{f1-1}$, that is, the signal detected by the sense coil 13a at the frequency $f_1$ after the first alternating magnetic field has been produced and the capsule medical device 3 has been delivered into the body cavity, contains signals with the frequency $f_1$ of the first alternating magnetic field output from the marker coil 4 and the second alternating magnetic field output from the magnetic-field generating device 41, as well as signals with the frequency $f_1$ of induced magnetic fields produced by the magnetic induction coil 5 in response to the first alternating magnetic field and the second alternating magnetic field (an induced magnetic field associated with the first alternating magnetic field and an induced magnetic field associated with the second alternating magnetic field).

Furthermore, $V_0^{f1-1}$, that is, the signal detected by the sense coil 13a at the frequency $f_1$ before the first alternating magnetic field is produced and the capsule medical device 3 is delivered into the body cavity, contains a signal with the frequency $f_1$ of the second alternating magnetic field output from the magnetic-field generating device 41.

Therefore, the signals at the frequency $f_1$ of the second alternating magnetic field are cancelled out by calculating the difference between them $(V^{f1-1} - V_0^{f1-1})$. For this reason, the first term (first detection-magnetic-field component) of each of the Expressions for $V_{m2}^{1}$ through $V_{m2}^{N}$ contains the signal with the frequency $f_1$ of the first alternating magnetic field, as well as the signals with the frequency $f_1$ of the induced magnetic field associated with the first alternating magnetic field and the induced magnetic field associated with the second alternating magnetic field.

In addition, the second term of each of the Expressions for $V_{m2}^{1}$ through $V_{m2}^{N}$ corresponds to magnetic-field information at the first position-calculating frequency $f_2$ (first detection-magnetic-field component). Here, of the second term $(V^{f2-1} \times \Sigma(V_0^{f1-N}) / \Sigma(V_0^{f2-N}) - V_0^{f2-1})$ of the Expression for $V_{m2}^{1}$, $V^{f2-1} \times \Sigma(V_0^{f1-N}) / \Sigma(V_0^{f2-N})$, that is, the signal detected by the sense coil 13a at the frequency $f_2$ after the first alternating magnetic field has been produced and the capsule medical device 3 has been delivered into the body cavity, contains signals with the frequency $f_2$ of the first alternating magnetic field output from the marker coil 4 and the second alternating magnetic field output from the magnetic-field generating device 41, as well as signals with the frequency $f_2$ of induced magnetic fields produced by the magnetic induction coil 5 in response to the first alternating magnetic field and the second alternating magnetic field (an induced magnetic field associated with the first alternating magnetic field and an induced magnetic field associated with the second alternating magnetic field).

Furthermore, $V_0^{f2-1}$, that is, the signal detected by the sense coil 13a at the frequency $f_2$ before the first alternating magnetic field is produced and the capsule medical device 3 is delivered into the body cavity, contains a signal with the frequency $f_2$ of the second alternating magnetic field output from the magnetic-field generating device 41.

Therefore, the signals at the frequency $f_2$ of the second alternating magnetic field are cancelled out by calculating the difference between them ($V^{f2-1} \times \Sigma(V_0^{f1-N})/\Sigma(V_0^{f2-N})-V_0^{f2-1}$). For this reason, the second term (first detection-magnetic-field component) of each of the Expressions for $V_{m2}^1$ through $V_{m2}^N$ contains the signal with the frequency $f_2$ of the first alternating magnetic field, as well as the signals with the frequency $f_2$ of the induced magnetic field associated with the first alternating magnetic field and the induced magnetic field associated with the second alternating magnetic field.

Here, the signals with the frequencies $f_1$ and $f_2$ of the induced magnetic field associated with the first alternating magnetic field have the characteristic that they differ from each other in the magnitude relationship of intensity with respect to the first alternating magnetic field and that they have substantially the same absolute value of the intensity. On the other hand, the signals with the frequencies $f_1$ and $f_2$ of the first alternating magnetic field have the same level of signal intensity because they have been subjected to the operation of making the gain of the signal at the frequency $f_1$ of each of the sense coils 13a substantially the same as the gain of the signal at $f_2$, as described above. As a result, when the difference between the first term and the second term of each of the Expressions for $V_{m2}^1$ through $V_{m2}^N$, that is, the difference between the single set of first detection-magnetic-field components is calculated, the signals of the first alternating magnetic field are further cancelled out, whereas the signals of the induced magnetic field associated with the first alternating magnetic field and the induced magnetic field associated with the second alternating magnetic field remain, without being cancelled out.

In this manner, the signals of the first alternating magnetic field and the signals of the second alternating magnetic field are canceled out by calculating the difference between the absolute values of magnetic-field intensity at the single set of first position-calculating frequencies $f_1$ and $f_2$, which are substantially the same frequency away from the resonance frequency $f_0$, with the first position-calculating frequencies $f_1$ and $f_2$ being on either side of the resonance frequency $f_0$. As a result, the signals of the induced magnetic fields produced by the first alternating magnetic field and the second alternating magnetic field (the induced magnetic fields associated with the first and second alternating magnetic fields) can be extracted easily.

.

The position/direction analyzing section 22 calculates the position and direction of the magnetic induction coil 5 through iterated arithmetic operations from $V_{m2}^1$, $V_{m2}^2$ ,...., $V_{m2}^N$ obtained in the extraction/calculation section (step S53).

The calculated position and direction of the magnetic induction coil 5 are sent to the control circuit 28 for display on the display device 8 (step S54) and stored in the second memory 23 (step S55).

Furthermore, in the extraction/calculation section, the signal from each of the sense coils 13a for calculating the position of the marker coil 4 is extracted from the Expressions shown below (step S56).

$$V_{m1}^1 = (V^{f1-1}-V_0^{f1-1}) + (V^{f2-1} \times \Sigma(V_0^{f1-N})/\Sigma(V_0^{f2-N})-V_0^{f2-1})$$

$$V_{m1}^2 = (V^{f1-2}-V_0^{f1-2}) + (V^{f2-2} \times \Sigma(V_0^{f1-N})/\Sigma(V_0^{f2-N})-V_0^{f2-2})$$

$$\cdots$$

$$V_{m1}^N = (V^{f1-N}-V_0^{f1-N}) + (V^{f2-N} \times \Sigma(V_0^{f1-N})/\Sigma(V_0^{f2-N})-V_0^{f2-N})$$

In this case, the first terms of the Expressions for $V_{m1}^1$ through $V_{m1}^N$ correspond to magnetic-field information at the first position-calculating frequency $f_1$ (first detection-magnetic-field components). Here, as described above, the first term $(V^{f1-1}-V_0^{f1-1})$ of the Expression for $V_{m1}^1$, that is, the signal detected by the sense coil 13a at the frequency $f_1$, contains a signal with the frequency $f_1$ of the first alternating magnetic field output from the marker coil 4, as well as signals with the frequency $f_1$ of induced magnetic fields produced by the magnetic induction coil 5 in response to the first alternating magnetic field and the second alternating magnetic field (an induced magnetic field associated with the first alternating magnetic field and an induced magnetic field associated with the second alternating magnetic field). In short, the signals at the frequency $f_1$ of the second alternating magnetic field output from the magnetic-field generating device 41 are cancelled out.

In addition, the second term of each of the Expressions for $V_{m1}^1$ through $V_{m1}^N$ corresponds to magnetic-field information at the first position-calculating frequency $f_2$ (first detection-magnetic-field component). Here, the second term $(V^{f2-1} \times \Sigma(V_0^{f1-N})/\Sigma(V_0^{f2-N})-V_0^{f2-1})$ of the Expression for $V_{m1}^1$, that is, the signal detected by the sense coil 13a at the frequency $f_2$, contains a signal with the frequency $f_2$ of the first alternating magnetic field output from the marker coil 4, as well as signals with the frequency $f_2$ of induced magnetic fields produced by the magnetic induction coil 5 in response to the first alternating magnetic field and the second alternating magnetic field (an induced magnetic field associated with the first alternating magnetic field and an induced magnetic field associated with the second alternating magnetic field). In short, the signals at the frequency $f_2$ of the second alternating magnetic field output from the magnetic-field generating device 41 are canceled out.

Here, the signals at the frequencies $f_1$ and $f_2$ of the induced magnetic field associated with the first alternating magnetic field and the induced magnetic field associated with the second alternating magnetic field have the characteristic that they differ from each other in the magnitude relationship of intensity with respect to the first alternating magnetic field and that they have substantially the same absolute value of the intensity. As a result, when the sum of the first term and the second term of each of the Expressions for $V_{m1}^1$ through $V_{m1}^N$, that is, the sum of the single set of first detection-magnetic-field components is calculated, the signals of the induced magnetic fields associated with the first and second alternating magnetic fields are further cancelled out, whereas the signals of the first alternating magnetic field remain, without being cancelled out.

In this manner, the signals of the second alternating magnetic field and the signals of the induced magnetic fields associated with the first and second alternating magnetic fields are canceled out by calculating the difference between the absolute value of magnetic-field intensity at the first position-calculating frequency $f_1$ extracted when the first alternating magnetic field is produced and the absolute value of magnetic-field intensity at the first position-calculating frequency $f_1$ extracted before the first alternating magnetic field is produced, as well as the sum of the differences between the absolute value of magnetic-field intensity at the first position-calculating frequency $f_2$ extracted when the first alternating magnetic field is produced and the absolute value of magnetic-field intensity at the first position-calculating frequency $f_2$ extracted before the first alternating magnetic field is produced. As a result, the signals of the first alternating magnetic field can be extracted easily.

The position/direction analyzing section 22 calculates the position and the direction of the marker coil 4 from $V_{m1}^1$, $Vm1^2$, ... $V_{m1}^N$ obtained in the extraction/calculation section 30 (step S57). Data on the calculated position and direction of the marker coil 4 is sent to the control circuit 28 and is then displayed on the display device 8 (step S58). Thereafter, the data on the calculated position and direction are accumulated in the second memory 23 (step S59).

Then, it is checked whether or not a command for terminating position detection has been input on the input device 26 (step S60), and if a command has been input, generation of a trigger signal from the trigger generator 31 is terminated to stop the operation of the position detection system 1 (step S61). On the other hand, if no termination command has been input, the flow returns to step S43 to continue position detection.

In this case, for the initial values for iterated arithmetic operations of the positions and directions of the marker coil 4 and the magnetic induction coil 5, the calculation results of the positions and the directions of the marker coil 4 and the magnetic induction coil 5 that have previously been calculated and stored in the second memory 23 are used. By doing so, the convergence time of iterated arithmetic operations can be reduced to calculate the positions and the directions in a shorter period of time.

As described above, according to the position detection system 40 of this embodiment and the position detection method using the system 40, at least one of the positions and the directions of the endoscope apparatus 2 and the capsule medical device 3 can be calculated simultaneously with high precision, even if the endoscope apparatus 2 having the marker coil 4 that produces a magnetic field by means of an external power supply and the capsule medical device 3 having the magnetic induction coil 5 coexist. In addition to the first alternating magnetic field, the second alternating magnetic field also produces an induced magnetic field from the magnetic induction coil 5, and therefore the intensity of the induced magnetic field can be increased.

Although the magnetic induction apparatus 101 is assumed to produce a rotating magnetic field in this embodiment, this method is not the only available one. Alternatively, the magnetic induction apparatus 101 may be made to produce a gradient magnetic field, which may then guide the capsule medical device 3 by a magnetic attraction force produced in the permanent magnet 150 of the capsule medical device 3.

[Third embodiment]

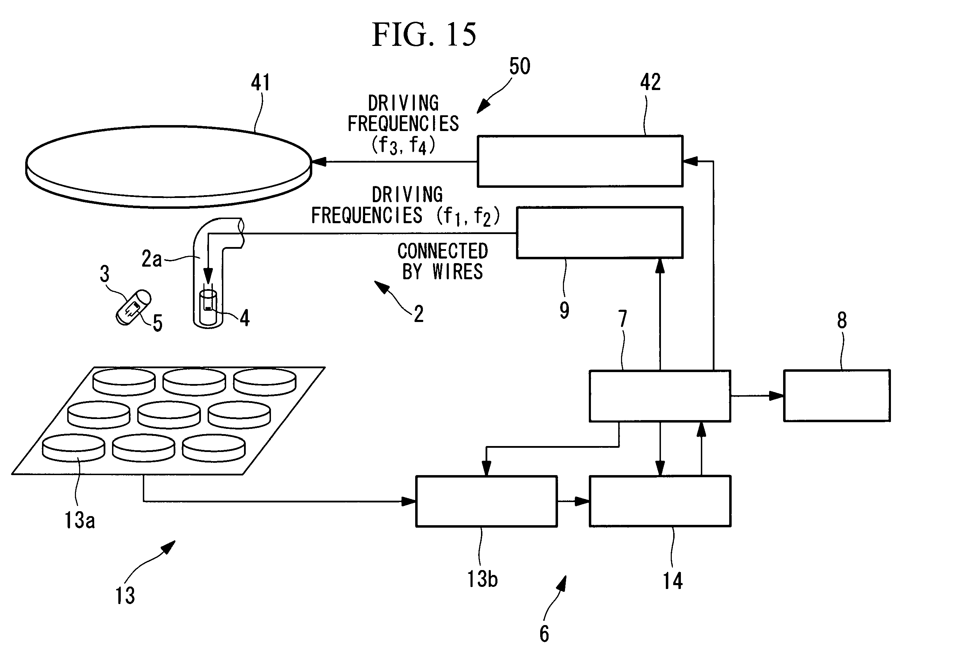

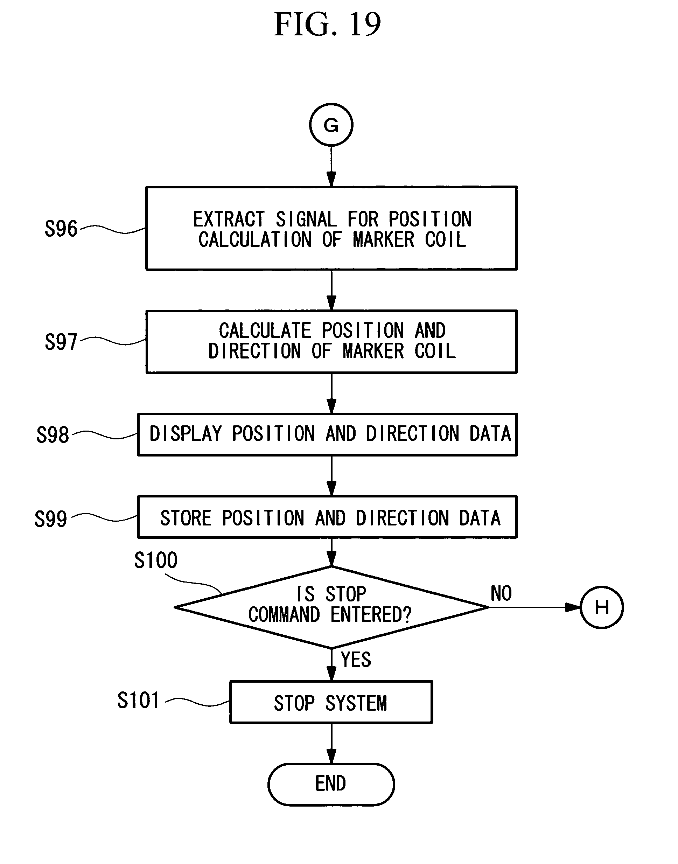

A position detection system 50 according to a third embodiment of the present invention will now be described with reference to Figs. 15 to 19.

In the description of this embodiment, the same components as those of the position detection system 40 according to the first embodiment are denoted by the same reference numerals, and thus an explanation thereof will be omitted.

As shown in Fig. 15, the position detection system 50 according to this embodiment differs from the position detection system 40 according to the second embodiment in that the frequencies of the second alternating magnetic field produced from the magnetic-field generating device 41 are a single set of second position-calculating frequencies $f_3$ and $f_4$, which differ from the frequencies $f_1$ and $f_2$ of the first alternating magnetic field.

In order to detect the positions and the directions of the marker coil 4 at the tip of the endoscope apparatus 2 and the magnetic induction coil 5 in the capsule medical device 3 by using the position detection system 50 according to this embodiment, waveform data of the produced first and second alternating magnetic fields is generated and is stored in the waveform data memories 10 and 43, and then calibration is carried out with the capsule medical device 3 being disposed outside the working region.

When the resonance frequency $f_0$ of the magnetic induction coil 5 is input via the input device 26, the control circuit 28 sets, as frequencies of the first alternating magnetic field to be produced from the marker coil 4, a single set of first position-calculating frequencies $f_1$ and $f_2$ that are away from the input resonance frequency $f_0$ by substantially equal frequencies, with the first position-calculating frequencies $f_1$ and $f_2$ being on either side of the resonance frequency $f_0$. In addition, the control circuit 28 sets, as second position-calculating frequencies of the second alternating magnetic field to be produced from the magnetic-field generating device 41, a single set of frequencies $f_3$ and $f_4$ that are away from the resonance frequency $f_0$ by substantially equal frequencies, with the frequencies $f_3$ and $f_4$ being on either side of the resonance frequency $f_0$, and that differ from the frequencies $f_1$ and $f_2$. Thereafter, when the control circuit 28 transfers the set first position-calculating frequencies $f_1$ and $f_2$ and the second position-calculating frequencies $f_3$ and $f_4$ to the waveform-data generator 27, generation of a magnetic-field waveform starts.

Because not only is the first alternating magnetic field produced from the marker coil 4 but also the second alternating magnetic field is produced from the magnetic-field generating device 41, items of data on the generated magnetic field waveforms are transferred to the waveform data memory 10 of the marker-driving circuit 9 and the waveform data memory 43 of the magnetic-field-generating-device driving circuit 42, respectively.

In the waveform-data generator 27, a magnetic-field waveform to be produced from the marker coil 4 based on the transferred single set of first position-calculating frequencies $f_1$ and $f_2$ is calculated using Expression (1) shown below.

$$B_{m1} = B_1 \times \sin(2\pi f_1 t) + B_2 \times \sin(2\pi f_2 t) \quad \dots \quad (1)$$

where $B_1$ and $B_2$ are set in accordance with the characteristics of the sense coils 13a so that the magnetic-field components at the frequencies $f_1$ and $f_2$ exhibit the same level of signal intensity when detected by the sense coils 13a. ($B_1$ and $B_2$ are set so that $B_1 \times f_1 = B_2 \times f_2$ if the sense coils 13a are ideal coils. Alternatively, the frequency characteristics of the sense coils 13a may be pre-measured to set $B_1$ and $B_2$ in accordance with the pre-measured frequency characteristics.)

Also in the waveform-data generator 27, a magnetic-field waveform to be produced from the magnetic-field generating device 41 based on the transferred single set of second position-calculating frequencies $f_3$ and $f_4$ is calculated using Expression (2') below.

$$B_G = B_3 \times \sin(2\pi f_3 t) + B_4 \times \sin(2\pi f_4 t) \quad \dots \quad (2')$$

Thereafter, data on the calculated magnetic-field waveform $B_{m1}$ is transferred to the marker-driving circuit 9 and is then stored in the waveform data memory 10. Furthermore, data on the calculated magnetic-field waveform $B_G$ is transferred to the magnetic-field-generating-device driving circuit 42 and is then stored in the waveform data memory 43.

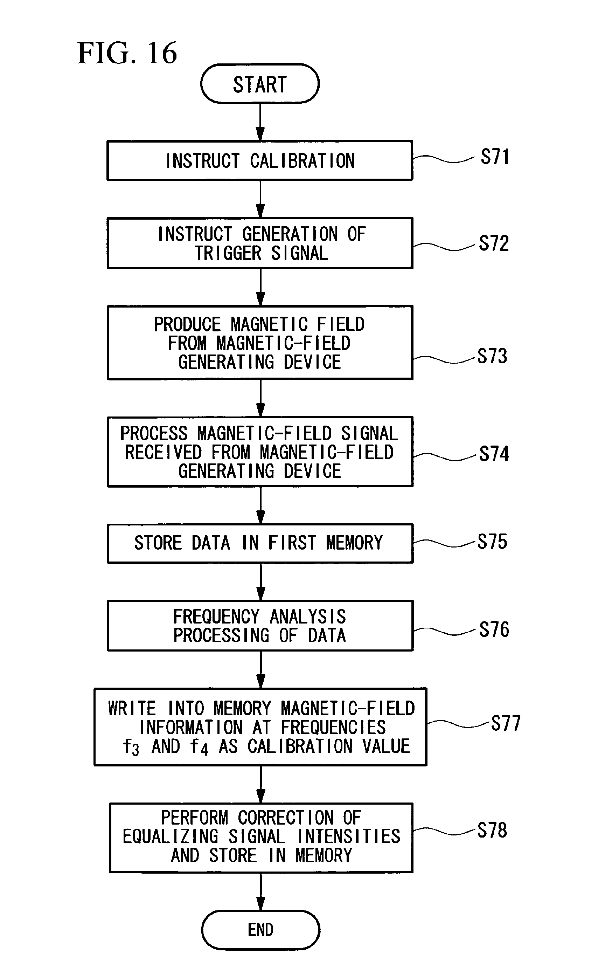

As shown in Fig. 16, calibration starts when a calibration command is input via the input device 26 while the tip of the inserting section 2a of the endoscope apparatus 2 is disposed in the body cavity and the capsule medical device 3 is not disposed in the body cavity (step S71). The control circuit 28 instructs the trigger generator 31 to produce a trigger signal for the magnetic-field-generating-device driving circuit 42. By doing so, a trigger signal is issued from the trigger generator 31 (step S72).

Based on the waveform data stored in the waveform data memory 43, the magnetic-field-generating-device driving circuit 42 that has received the trigger signal sequentially generates magnetic-field-generation driving signals in synchronization with the clock signal from the clock 29 and outputs them to the magnetic-field generating device 41. The magnetic-field generating device 41 produces the second alternating magnetic field based on the input magnetic-field-generation driving signals (step S73).

The receiving circuit 13b receives a magnetic-field signal associated with the second alternating magnetic field from the magnetic-field generating device 41 detected by each of the sense coils 13a; performs low-pass filtering, amplification, and band-pass filtering; and then performs A/D conversion in synchronization with the clock signal from the clock 32 (step S74).

The magnetic-field signal that has been subjected to A/D conversion is stored in the first memory 19 of the position-calculating section 14 (step S75). Thereafter, it is determined whether or not a number of items of data required to perform frequency analysis processing are accumulated in the first memory 19, and if the required number of items of data are accumulated, frequency analysis processing is performed by the FFT-processing circuit 20 (step S76).

Based on the result of frequency analysis processing, the frequency-selecting section 24 extracts only the magnetic-field information at the second position-calculating frequencies $f_3$ and $f_4$ of the second alternating magnetic field produced from the magnetic-field generating device 41 and stores it in the third memory 25 in association with the frequencies $f_3$ and $f_4$ (step S77).

Let the signal intensities of the stored magnetic-field information at the second position-calculating frequencies $f_3$ and $f_4$ at this time be respectively represented as $V_0^{f3-1}$, $V_0^{f3-2}$, ... $V_0^{f3-N}$, $V_0^{f4-1}$, $V_0^{f4-2}$, ... $V_0^{f4-N}$, where superscripts $f_3$ and $f_4$ indicate frequency components and the subsequent superscripts 1, 2, ..., N indicate the numbers of the sense coils 13a. Furthermore, the term "magnetic-field information" means the absolute value of the result of FFT processing. The magnetic-field information at these second position-calculating frequencies $f_3$ and $f_4$ is stored in the third memory 25 as calibration values.

Here, the signal intensities at the frequency $f_3$ and the signal intensities at the frequency $f_4$ detected by all the sense coils 13a are corrected.

More specifically, the sum $\Sigma(V_0^{f3-N})$ of the signal components at the frequency $f_3$ detected by all the sense coils 13a and the sum $\Sigma(V_0^{f4-N})$ of the signal components at the frequency $f_4$ detected by all the sense coils 13a are obtained first. Then, the ratio of the sums of the signal components $\Sigma(V_0^{f3-N})/\Sigma(V_0^{f4-N})$ is obtained as a correction factor.

Subsequently, $V_0^{f4-1}$, $V_0^{f4-2}$ ..., $V_0^{f4-N}$ are replaced as shown below using the obtained correction factor by overwriting the third memory 25.

$$V_0^{f4-1} \text{ is replaced by } V_0^{f4-1} \times \Sigma(V_0^{f3-N})/\Sigma(V_0^{f4-N}).$$

$$V_0^{f4-2} \text{ is replaced by } V_0^{f4-2} \times \Sigma(V_0^{f3-N})/\Sigma(V_0^{f4-N}).$$

$$...$$

$$V_0^{f4-N} \text{ is replaced by } V_0^{f4-N} \times \Sigma(V_0^{f3-N})/\Sigma(V_0^{f4-N}).$$

Furthermore, the correction factor $\Sigma(V_0^{f3-N})/\Sigma(V_0^{f4-N})$ is also stored in the third memory 25 (step S78).

By doing so, $V_0^{f3-1}$ and $V_0^{f4-1}$ ($V_0^{f4-1} \times (V_0^{f3-N})/\Sigma(V_0^{f4-N})$ as a result of replacement) stored in the third memory 25 have substantially the same values. In other words, an operation is carried out for making the gain for the signal at the frequency $f_3$ from each of the sense coils 13a substantially the same as the gain for the signal at the frequency $f_4$.

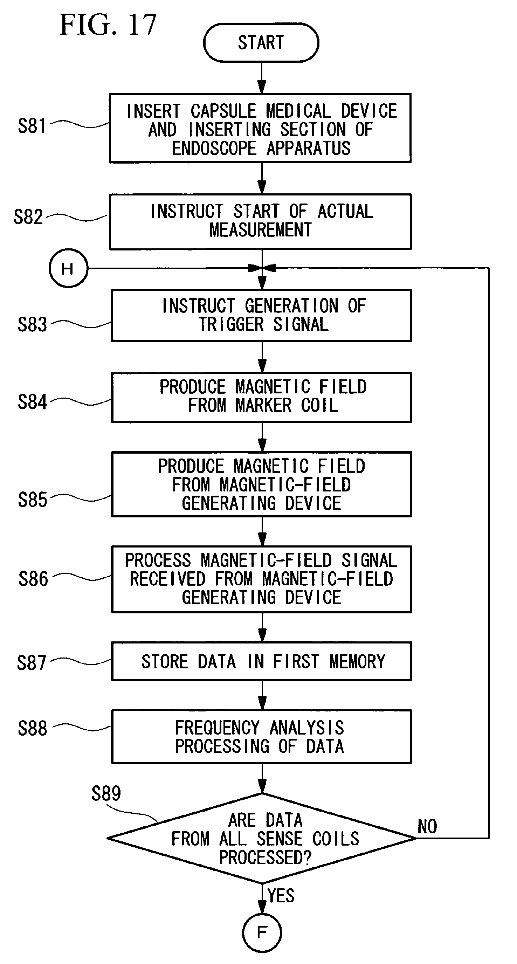

Next, actual measurement starts when a command for starting actual measurement is entered on the input device 26 (step S82) with the endoscope apparatus 2 and the capsule medical device 3 being disposed in the body cavity (step S81), as shown in Figs. 17 to 19.

The control circuit 28 instructs the trigger generator 31 to produce a trigger signal for the marker-driving circuit 9 and the magnetic-field-generating-device driving circuit 42, and the trigger generator 31 produces a trigger signal (step S83).

The marker-driving circuit 9 sequentially generates magnetic-field-generation driving signals in synchronization with the clock signal based on the waveform data stored in the waveform data memory 10 and outputs them to the marker coil 4. The marker coil 4 produces the first alternating magnetic field based on the input magnetic-field-generation driving signals (step S84).

Furthermore, based on the waveform data stored in the waveform data memory 43, the magnetic-field-generating-device driving circuit 42 sequentially generates magnetic-field-generation driving signals in synchronization with the clock signal and outputs them to the magnetic-field generating device 41. The magnetic-field generating device 41 produces the second alternating magnetic field based on the input magnetic-field-generation driving signals (step S85).

The receiving circuit 13b applies low-pass filtering, amplification, and band-pass filtering to a magnetic-field signal associated with the first alternating magnetic field from the marker coil 4 and to a magnetic-field signal associated with the second alternating magnetic field from the magnetic-field generating device 41, i.e., the magnetic-field signals detected by each of the sense coils 13a, and then performs A/D conversion in synchronization with the clock signal from the clock 32 (step S86).

The magnetic-field signals that have been subjected to A/D conversion are stored in the first memory 19 of the position-calculating section 14 (step S87).

Then, it is determined whether or not a number of items of data required to perform frequency analysis processing are accumulated in the first memory 19, and if the required number of items of data are accumulated, the FFT-processing circuit 20 reads out signal data from the first memory 19 and carries out frequency analysis processing (step S88). Thereafter, it is determined whether or not the data from all the sense coils 13a have been subjected to this frequency analysis processing (step S89). If data from all sense coils 13a have not been processed, steps S83 to S88 are repeated.

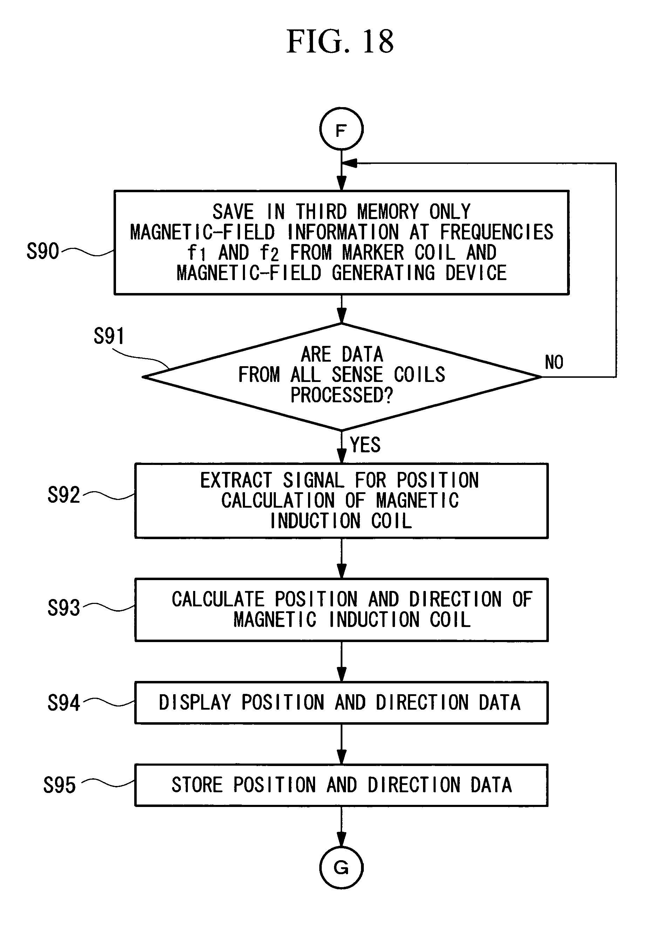

When the data from all the sense coils 13a have been subjected to frequency analysis processing, the frequency-selecting section 24 extracts, based on the result of processing, only the magnetic-field information at the first position-calculating frequencies $f_3$ and $f_4$ of the first alternating magnetic field produced from the marker coil 4 and the second alternating magnetic field produced from the magnetic-field generating device 41, as shown in Fig. 18, and stores it in the third memory 25 in association with the frequencies $f_3$ and $f_4$ (step S90). This processing is applied to the magnetic-field signals from all the sense coils 13a (step S91).

In the extraction/calculation section 30, the signal from each of the sense coils 13a for calculating the position of the magnetic induction coil 5 is extracted from the Expressions shown below (step S92).

$$V_{m2}{}^1 = (V^{f3-1} - V_0{}^{f3-1}) - (V^{f4-1} \times \Sigma (V_0{}^{f3-N}) / \Sigma (V_0{}^{f4-N}) - V_0{}^{f4-1})$$

$$V_{m2}{}^2 = (V^{f3-2} - V_0{}^{f3-2}) - (V^{f4-2} \times \Sigma (V_0{}^{f3-N}) / \Sigma (V_0{}^{f4-N}) - V_0{}^{f4-2})$$

$$\cdots$$

$$V_{m2}{}^N = (V^{f3-N} - V_0{}^{f3-N}) - (V^{f4-N} \times \Sigma (V_0{}^{f3-N}) / \Sigma (V_0{}^{f4-N}) - V_0{}^{f4-N})$$

In this case, the first terms of the Expressions for $V_{m2}{}^1$ through $V_{m2}{}^N$ correspond to magnetic-field information at the second position-calculating frequency $f_3$ (second detection-magnetic-field components). Also, the second terms of the Expressions for $V_{m2}{}^1$ through $V_{m2}{}^N$ correspond to magnetic-field information at the second position-calculating frequency $f_4$ (second detection-magnetic-field components).

In this manner, the signals of the second alternating magnetic field can be canceled out by calculating the difference between the absolute values of magnetic-field intensity at the single set of second position-calculating frequencies $f_3$ and $f_4$, which are substantially the same frequency away from the resonance frequency $f_0$, with the second position-calculating frequencies $f_3$ and $f_4$ being on either side of the resonance frequency $f_0$. As a result, the signals of the induced magnetic field produced by the second alternating magnetic field (the induced magnetic field associated with the second alternating magnetic field) can be extracted easily.

The position/direction analyzing section 22 calculates the position and direction of the magnetic induction coil 5 through iterated arithmetic operations from $V_{m2}{}^1$, $V_{m2}{}^2$ ,..., $V_{m2}{}^N$ obtained in the extraction/calculation section (step S93).

The calculated position and direction of the magnetic induction coil 5 are sent to the control circuit 28 for display on the display device 8 (step S94) and stored in the second memory 23 (step S95).

Furthermore, in the extraction/calculation section, the signal from each of the sense coils 13a for calculating the position of the marker coil 4 is extracted from the Expressions shown below (step S96).

$$V_{m1}{}^1 \;=\; V^{f1-1} \;+\; V^{f2-1}$$

$$V_{m1}{}^2 \;=\; V^{f1-2} \;+\; V^{f2-2}$$

$$\ldots$$

$$V_{m1}{}^N \;=\; V^{f1-N} \;+\; V^{f2-N}$$

In this case, the first terms of the Expressions for $V_{m1}{}^1$ through $V_{m1}{}^N$ correspond to magnetic-field information at the first position-calculating frequency $f_1$ (first detection-magnetic-field components). Here, the first term of the Expression for $V_{m1}{}^1$, that is, the signal detected by the sense coil 13a at the frequency $f_1$, contains a signal with the frequency $f_1$ of the first alternating magnetic field output from the marker coil 4, as well as a signal with the frequency $f_1$ of an induced magnetic field produced by the magnetic induction coil 5 in response to the first alternating magnetic field (an induced magnetic field associated with the first alternating magnetic field).
In addition, the second term of each of the Expressions for $V_{m1}{}^1$ through $V_{m1}{}^N$ corresponds to magnetic-field information at the first position-calculating frequency $f_2$ (first detection-magnetic-field component). Here, the second term of the Expression for $V_{m1}{}^1$, that is, the signal detected by the sense coil 13a at the frequency $f_2$, contains a signal with the frequency $f_2$ of the first alternating magnetic field output from the marker coil 4, as well as a signal with the frequency $f_2$ of an induced magnetic field produced by the magnetic induction coil 5 in response to the first alternating magnetic field (an induced magnetic field associated with the first alternating magnetic field).

As described above, in the process of calculating a magnetic-field waveform to be generated from the marker coil 4, $B_1$ and $B_2$ are set so as to exhibit the same level of signal intensity when the magnetic-field components at the frequencies $f_1$ and $f_2$ are detected by the sense coils 13a. Therefore, the signals with the frequencies $f_1$ and $f_2$ of the induced magnetic field associated with the first alternating magnetic field have the characteristic that they differ from each other in the magnitude relationship of intensity with respect to the first alternating magnetic field and that they have substantially the same absolute value of the intensity. As a result, when the sum of the first term and the second term of each of the Expressions for $V_{m1}{}^1$ through $V_{m1}{}^N$, that is, the sum of the single set of first detection-magnetic-field components is calculated, the signals of the induced magnetic field associated with the first alternating magnetic field are cancelled out.
In this manner, the signals of the induced magnetic field associated with the first alternating magnetic field can be canceled out by calculating the sum of the single set of the absolute value of the magnetic-field intensity at the first position-calculating frequencies $f_1$ and the absolute value of the magnetic-field intensity at the first position-calculating frequency $f_2$, which are substantially the same frequency away from the resonance frequency $f_0$, with the first position-calculating frequencies $f_1$ and $f_2$ being on either side of the resonance frequency $f_0$. As a result, the signals of the first alternating magnetic field can be extracted easily.

The position/direction analyzing section 22 calculates the position and the direction of the marker coil 4 from $V_{m1}{}^1$, $V_{m1}{}^2$, ... $V_{m1}{}^N$ obtained in the extraction/calculation section 30 (step S97).
Data on the calculated position and direction of the marker coil 4 is sent to the control circuit 28 and is then displayed on the display device 8 (step S98). Thereafter, the data on the calculated position and direction are accumulated in the second memory 23 (step S99).

Then, it is checked whether or not a command for terminating position detection has been input on the input device 26 (step S100), and if a command has been input, generation of a trigger signal from the trigger generator 31 is terminated to stop the operation of the position detection system 1 (step S101). On the other hand, if no termination command has been input, the flow returns to step S43 to continue position detection.

In this case, for the initial values for iterated arithmetic operations of the positions and directions of the marker coil 4 and the magnetic induction coil 5, the calculation results of the positions and the directions of the marker coil 4 and the magnetic induction coil 5 that have previously been calculated and stored in the second memory 23 are used. By doing so, the convergence time of iterated arithmetic operations can be reduced to calculate the positions and the directions in a shorter period of time.

As described above, according to the position detection system 50 of this embodiment and the position detection method using the system 50, at least one of the positions and the directions of the endoscope apparatus 2 and the capsule medical device 3 can be calculated simultaneously with high precision, even if the endoscope apparatus 2 having the marker coil 4 that produces a magnetic field by means of an external power supply and the capsule medical device 3 having the magnetic induction coil 5 coexist. In addition, because it is easy to enhance the output of the second alternating magnetic field to be produced from the magnetic-field generating device 41 disposed outside the working region of the magnetic induction coil 5, the intensity of the induced magnetic field associated with the second alternating magnetic field can be increased.

This embodiment has been described assuming that the endoscope apparatus 2 includes a single marker coil 4. If the endoscope apparatus 2 includes a plurality of marker coils 4 that produce first alternating magnetic fields having a plurality of mutually different sets of first position-calculating frequencies, the following processing is performed.

The waveform-data generator 27 calculates magnetic-field waveforms to be produced from the plurality of marker coils 4. The magnetic fields to be produced are as follows.

First marker coil 4:

$$B_{m11} = B_{11} \times \sin(2\pi f_{11}t) + B_{21} \times \sin(2\pi f_{21}t)$$

Second marker coil 4

$$B_{m12} = B_{12} \times \sin(2\pi f_{12}t) + B_{22} \times \sin(2\pi f_{22}t)$$

$$...$$

$$\text{n-th marker coil 4}$$

$$B_{m1n} = B_{1n} \times \sin(2\pi f_{1n}t) + B_{22} \times \sin(2\pi f_{2n}t)$$

In the above Expressions, $B_{11}$ and $B_{21}$ are set in accordance with the characteristics of the sense coils 13a so that the magnetic-field components at the frequencies $f_{11}$ and $f_{21}$ exhibit the same level of signal intensity when detected by the sense coils 13a. ($B_{11}$ and $B_{21}$ are set so that $B_{11} \times f_{11} = B_{21} \times f_{21}$ if the sense coils 13a are ideal coils. Alternatively, the frequency characteristics of the sense coils 13a may be pre-measured to set $B_{11}$ and $B_{21}$ in accordance with the pre-measured frequency characteristics.) Thereafter, setting is carried out so that $B_{12}$, $B_{22}$, $f_{12}$, and $f_{22}$, ..., $B_{1n}$, $B_{2n}$, $f_{1n}$, and $f_{2n}$ exhibit the same relationships.

Furthermore, in actual measurement, the extraction/calculation section 30 extracts the signal from each of the sense coils 13a for performing one calculation of the first to n-th marker coils 4 based on the Expressions shown below.

$$V_{m11}^{1} = V^{f11-1} + V^{f21-1}, \quad V_{m11}^{2} = V^{f11-2} + V^{f21-2}, \quad ... \quad , \quad V_{m11}^{N} =$$

$$V^{f11-N} + V^{f21-N}$$

$$V_{m12}^{1} = V^{f12-1} + V^{f22-1}, \quad V_{m12}^{2} = V^{f12-2} + V^{f22-2}, \quad ... \quad , \quad V_{m12}^{N} =$$

$$V^{f12-N} + V^{f22-N}$$

$$...$$

$$V_{m1n}^{1} = V^{f1n-1} + V^{f2n-1}, \quad V_{m1n}^{2} = V^{f1n-2} + V^{f2n-2}, \quad ... \quad , \quad V_{m1n}^{N} =$$

$$V^{f1n-N} + V^{f2n-N}$$

Furthermore, the position/direction analyzing section 22 can be made to calculate the position and the direction of the first marker coil 4 from $V_{m11}^{1}$, $V_{m11}^{2}$, ..., $V_{m11}^{N}$ obtained in the extraction/calculation section 30 and to calculate the position and the direction of the n-th marker coil 4 from $V_{m1n}^{1}$, $V_{m1n}^{2}$, ..., $V_{m1n}^{N}$.

Alternatively, a case where a marker coil 4 having a plurality of mutually different sets of first position-calculating frequencies is provided in a plurality of endoscope apparatuses 2, instead of a plurality of marker coils 4 being provided in a single endoscope apparatus 2, can also be handled through the same processing.

[Fourth embodiment]

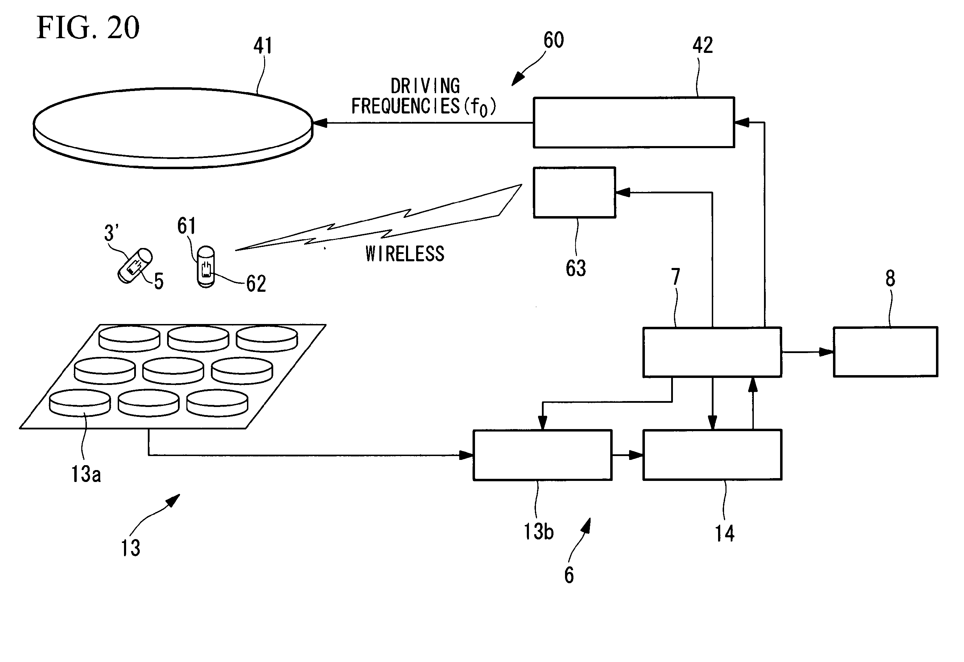

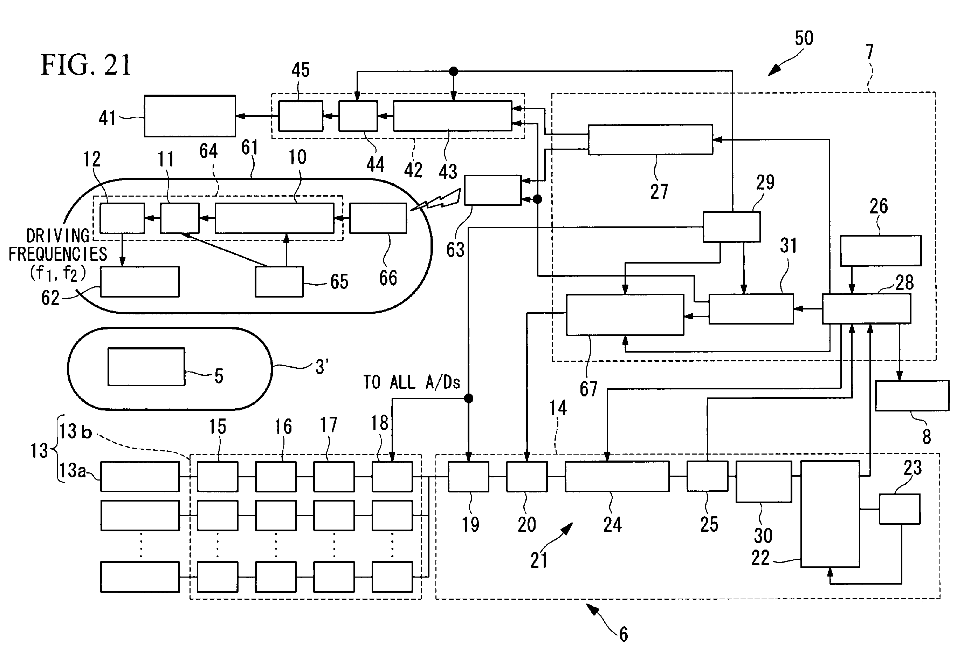

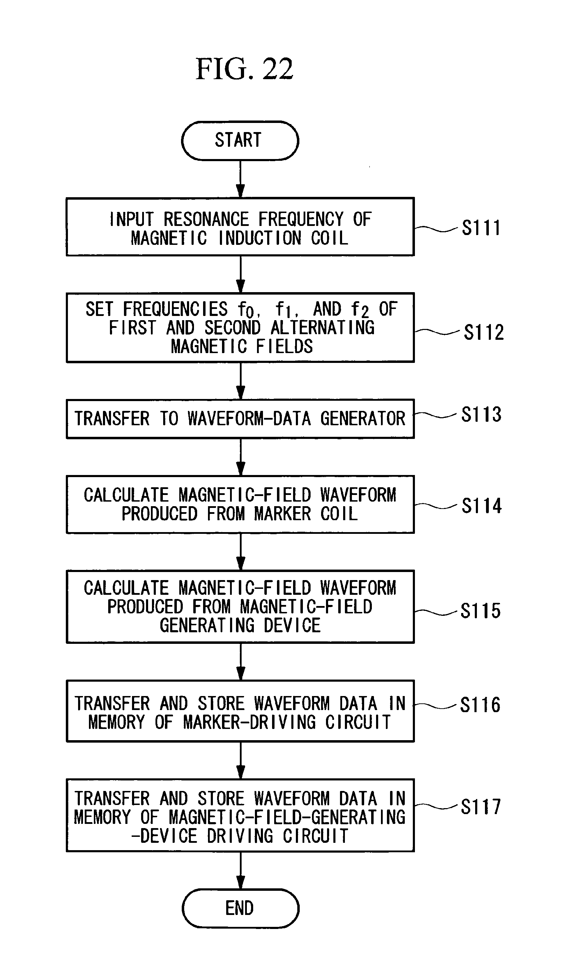

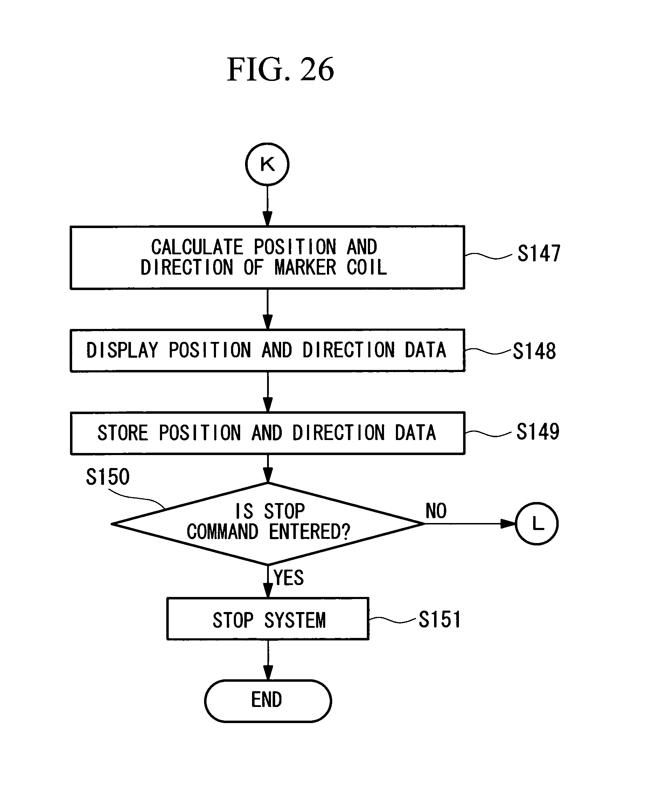

A position detection system 60 according to a fourth embodiment of the present invention will now be described with reference to Figs. 20 to 26.
In the description of this embodiment, the same components as those of the position detection system 40 according to the second embodiment are denoted by the same reference numerals, and thus an explanation thereof will be omitted.
As shown in Fig. 20, the position detection system 60 according to this embodiment differs from the position detection system 40 according to the above-described second embodiment in that a marker coil 62 disposed in a first capsule medical device 61 is employed in place of the marker coil 4 provided at the tip of the endoscope apparatus 2, a section 63 for transmitting a signal to the first capsule medical device 61 is provided, the magnetic induction coil 5 is disposed in a second capsule medical device 3', and the frequency of the second alternating magnetic field to be produced by the magnetic-field generating device 41 is different. The system 60 also differs from the system 40 in computational processing performed in the position-calculating section 14.
Furthermore, the position detection system 60 according to this embodiment includes in the control section 7 a read-out-timing generator 67 that instructs the FFT-processing circuit 20 of the position-calculating section 14 on the read-out timing of the magnetic-field signal used for frequency analysis based on a clock signal from the clock 29.
As shown in Fig. 21, the first capsule medical device 61 includes the marker coil 62, which produces the first alternating magnetic field having the first position-calculating frequencies $f_1$ and $f_2$; a marker-driving circuit 64 that drives the marker coil 62; a clock 65; a reception section 66; and a power supply (not shown in the figure). The marker-driving circuit 64 causes the marker coil 62 to produce the first alternating magnetic field according to a command signal that is wirelessly transmitted from the transmission section 63 and received by the reception section 66.
The above-described magnetic-field generating device 41 produces the second alternating magnetic field having the resonance frequency $f_0$ of the magnetic induction coil 5 in the second capsule medical device 3'.
In order to detect the positions and the directions of the marker coil 62 in the first capsule medical device 61 and the magnetic induction coil 5 in the second capsule medical device 3' using the position detection system 60 according to this embodiment, the waveform data of an alternating magnetic field to be produced is generated and stored in the waveform data memories 10 and 43 and then the read-out timing is set while the second capsule medical device 3' is not disposed in the working region.
Items of data on the generated magnetic field waveforms are transferred to the waveform data memory 10 of the marker-driving circuit 64 in the first capsule medical device 61 and the waveform data memory 43 of the magnetic-field-generating-device driving circuit 42, respectively.
Generation of a magnetic-field waveform starts when the resonance frequency $f_0$ of the magnetic induction coil 5 is entered on the input device 26, as shown in Fig. 22 (step S111). The control circuit 28 sets a single set of frequencies $f_1$ and $f_2$ that are away from the input resonance frequency $f_0$ by substantially equal frequencies, with the frequencies $f_1$ and $f_2$ being on either side of the resonance frequency $f_0$, as the first position-calculating frequencies $f_1$ and $f_2$ of the first alternating magnetic field to be produced from the marker coil 62 in the first capsule medical device 61. Furthermore, the control circuit 28 sets the resonance frequency $f_0$ as the second position-calculating frequency $f_0$ of the second alternating magnetic field to be produced from the magnetic induction coil 5 (step S112).
The control circuit 28 transfers the set frequencies $f_0$, $f_1$, and $f_2$ to the waveform-data generator 27 (step S113). In the waveform-data generator 27, the magnetic-field waveform to be produced from the marker coil 4 is calculated based on the transferred first position-calculating frequencies $f_1$ and $f_2$. The magnetic field to be produced is calculated based on the Expression (1) shown below (step S114).

$$B_{m1} = B_1 \times \sin(2\pi f_1 t) + B_2 \times \sin(2\pi f_2 t) \quad ... \quad (1)$$

where $B_1$ and $B_2$ are set in accordance with the characteristics of the sense coils 13a so that the magnetic-field components at the frequencies $f_1$ and $f_2$ exhibit the same level of signal intensity when detected by the sense coils 13a. ($B_1$ and $B_2$ are set so that $B_1 \times f_1 = B_2 \times f_2$ if the sense coils 13a are ideal coils. Alternatively, the frequency characteristics of the sense coils 13a may be pre-measured to set $B_1$ and $B_2$ in accordance with the pre-measured frequency characteristics.)
Furthermore, the waveform-data generator 27 calculates a magnetic-field waveform to be produced from the magnetic-field generating device 41. The magnetic field to be produced is calculated based on the Expression (2") shown below (step S115).

$$B_G = B_3 \times \sin(2\pi f_0 t) \quad \ldots \quad (2'')$$

Data on the magnetic-field waveform $B_{m1}$ generated in the waveform-data generator 27 is transmitted from the transmission section 63 provided in the control section 7 to the reception section 66 provided in the first capsule medical device 61. Data on the magnetic field waveform that has been received in the reception section 66 is stored in the waveform data memory 10 (step S116). Furthermore, data on the magnetic-field waveform $B_G$ is stored in the waveform data memory 43 of the magnetic-field-generating-device driving circuit 42 (step S117).

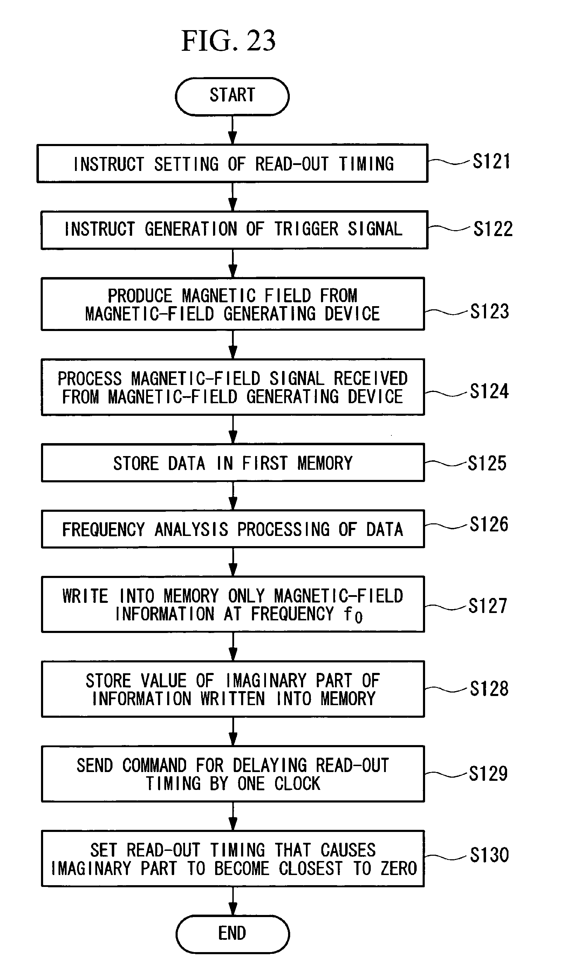

Setting of read-out timing in the read-out-timing generator 67 will be described with reference to Fig. 23. Setting of read-out timing starts when a command for setting the read-out timing is entered on the input device 26 with the first capsule medical device 61 being disposed in the body cavity and the second capsule medical device 3' not being disposed in the body cavity (step S121).

The control circuit 28 instructs the trigger generator 31 to produce a trigger signal for the magnetic-field-generating-device driving circuit 42 and the read-out-timing generator 67. By doing so, a trigger signal is issued from the trigger generator 31 (step S122).

Based on the data for the magnetic-field waveform $B_G$ stored in the waveform data memory 43, the magnetic-field-generating-device driving circuit 42 that has received the trigger signal sequentially generates magnetic-field-generation driving signals in synchronization with the clock signal from the clock 29 and outputs them to the magnetic-field generating device 41. The magnetic-field generating device 41 produces the second alternating magnetic field based on the input magnetic-field-generation driving signals (step S123).

The receiving circuit 13b receives a magnetic-field signal associated with the second alternating magnetic field from the magnetic-field generating device 41 detected by each of the sense coils 13a; performs low-pass filtering, amplification, and band-pass filtering; and then performs A/D conversion in synchronization with the clock signal from the clock 29 (step S124).

The magnetic-field signal that has been subjected to A/D conversion is stored in the first memory 19 of the position-calculating section 14 (step S125). Thereafter, it is determined whether or not a number of items of data required to perform frequency analysis processing are accumulated in the first memory 19, and if the required number of items of data are accumulated, frequency analysis processing is performed by the FFT-processing circuit 20 (step S126).

Based on the result of frequency analysis processing, the frequency-selecting section 24 extracts only the magnetic-field information at the second position-calculating frequency $f_0$ (second detection-magnetic-field component), which is the frequency of the second alternating magnetic field produced from the magnetic-field generating device 41, and stores it in the third memory 25 (step S127). Here, the magnetic-field information is the value of the imaginary part in the result of frequency analysis processing.

The control circuit 28 reads out the magnetic-field information stored in the third memory 25 and stores the value of the imaginary part in the internal memory (step S128). Then, the control circuit 28 sends to the read-out-timing generator 67 a command for delaying by one clock the read-out timing to be produced in the read-out-timing generator 67 (step S129).

Thereafter, while repeating steps S122 to S129, the control circuit 28 compares the imaginary part of the magnetic-field information stored in the third memory 25 with the imaginary part stored in the internal memory. The control circuit 28 sets, in the read-out-timing generator 67, the read-out timing that causes the value of the imaginary part in the result of the frequency analysis processing stored at step S128 to become closest to zero as the read-out timing used for actual measurement (step S130).

This completes the setting of the read-out timing. Thus, the imaginary part in the result of frequency analysis processing can be made independent of the magnetic-field information from the magnetic-field generating device 41.

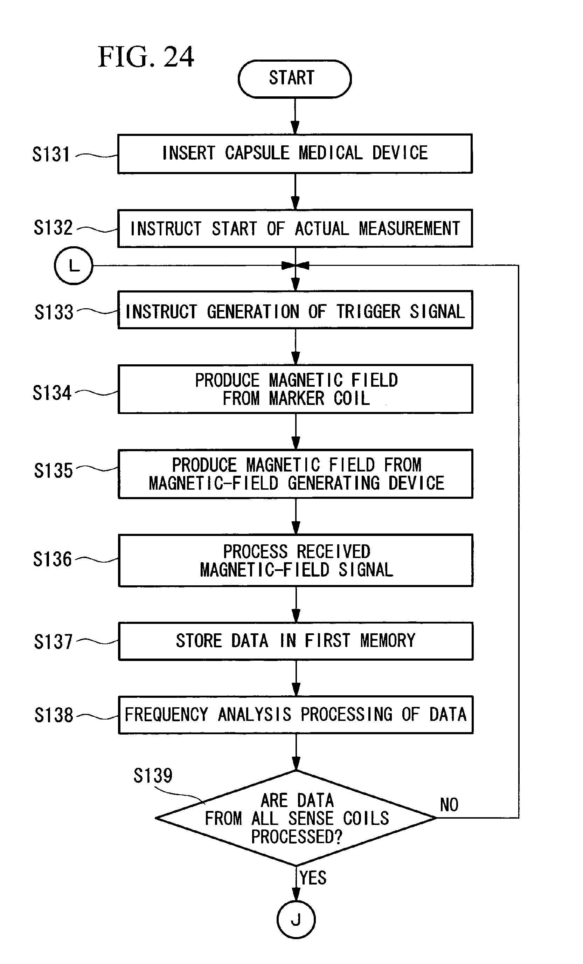

As shown in Fig. 24, actual measurement starts when a command for starting actual measurement is entered on the input device 26 (step S132) with the first and second capsule medical devices 61 and 3' being disposed in the body cavity (step S131).

The control circuit 28 instructs the trigger generator 31 to produce a trigger signal for the marker-driving circuit 64, the magnetic-field-generating-device driving circuit 42 ,and the read-out-timing generator 67, and the trigger generator 31 produces a trigger signal (step S133).

The marker-driving circuit 64 sequentially generates magnetic-field-generation driving signals in synchronization with the clock signal based on the waveform data stored in the waveform data memory 10 and outputs them to the marker coil 62. The marker coil 62 produces the first alternating magnetic field based on the input magnetic-field-generation driving signals (step S134).

Furthermore, the magnetic-field-generating-device driving circuit 42 sequentially generates magnetic-field-generation driving signals in synchronization with the clock signal based on the waveform data stored in the waveform data memory 43 and outputs them to the magnetic-field generating device 41. The magnetic-field generating device 41 produces the second alternating magnetic field with the input magnetic-field-generation driving signals (step S135).

The receiving circuit 13b applies low-pass filtering, amplification, and band-pass filtering to the magnetic-field signals, associated with the first alternating magnetic field from the marker coil 62 and the second alternating magnetic field from the magnetic-field generating device 41, detected by the sense coils 13a and then performs A/D conversion in synchronization with the clock signal from the clock 29 (step S136).

Each of the magnetic-field signals that have been subjected to A/D conversion is stored in the first memory 19 of the position-calculating section 14 (step S137). Then, it is determined whether or not a number of items of data required to perform frequency analysis processing are accumulated in the first memory 19, and if the required number of items of data are accumulated, the FFT-processing circuit 20 reads out signal data from the first memory 19 of the position-calculating section 14 based on the signal from the read-out-timing generator 67 and performs frequency analysis processing (step S138).

Thereafter, it is determined whether or not this frequency analysis processing has been applied to the data from all the sense coils 13a (step S139), and if data from all the sense coils 13a have not been processed, steps S133 to S138 are repeated.

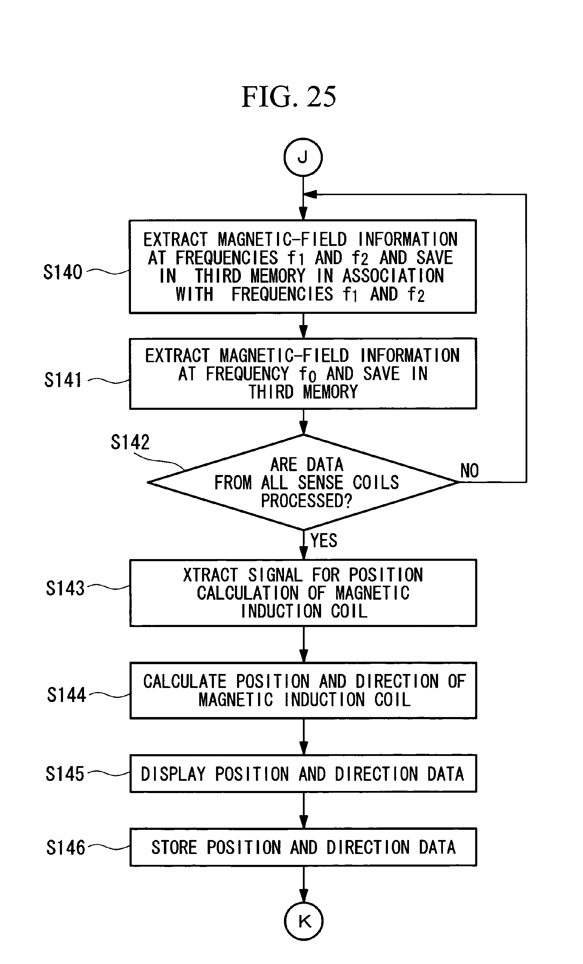

When the data from all the sense coils 13a have been subjected to frequency analysis processing, the frequency-selecting section 24 extracts, based on the result of processing, only the magnetic-field information at the first position-calculating frequencies $f_1$ and $f_2$ of the first alternating magnetic field produced from the marker coil 4 and stores it in the third memory 25 in association with the frequencies $f_1$ and $f_2$, as shown in Fig. 25 (step S140). Furthermore, the frequency-selecting section 24 extracts only the magnetic-field information at the second position-calculating frequency $f_0$ of the second alternating magnetic field produced from the magnetic-field generating device 41 and stores it in the third memory 25 (step S141). This processing is applied to the magnetic-field signals from all the sense coils 13a (step S142).

Of the magnetic-field information stored in the third memory 25, the position/direction analyzing section 22 reads out the imaginary part in the result of frequency analysis processing (second detection-magnetic-field component) (step S143) and, based on the imaginary part, calculates the position and the direction of the magnetic induction coil 5 (step S144).

Because the imaginary part in the result of frequency analysis has a phase shifted by n/2 relative to that of the second alternating magnetic field, the signal of the induced magnetic field produced by the second alternating magnetic field can be extracted by extracting this imaginary part.

The calculated position and direction of the magnetic induction coil 5 are sent to the control circuit 28, displayed on the display device 8 (step S145), and stored in the second memory 23 (step S146).

In the extraction/calculation section 30, the signal from each of the sense coils 13a for calculating the position of the marker coil 4 is extracted from the Expressions shown below.

$$V_{m1}{}^1 = V^{f1-1} + V^{f2-1}$$

$$V_{m1}{}^2 = V^{f1-2} + V^{f2-2}$$

$$\cdots$$

$$V_{m1}{}^N = V^{f1-N} + V^{f2-N}$$

In this case, the first terms of the Expressions for $V_{m1}{}^1$ through $V_{m1}{}^N$ correspond to magnetic-field information at the first position-calculating frequency $f_1$ (first detection-magnetic-field components). Here, the first term of the Expression for $V_{m1}{}^1$, that is, the signal detected by the first sense coil 13a at the frequency $f_1$ contains a signal with the frequency $f_1$ of the first alternating magnetic field output from the marker coil 4, as well as a signal with the frequency $f_1$ of the induced magnetic field generated by the magnetic induction coil 5 in response to the first alternating magnetic field from the marker coil 4 (induced magnetic field associated with the first alternating magnetic field).

Furthermore, the second terms of the Expressions for $V_{m1}{}^1$ through $V_{m1}{}^N$ correspond to magnetic-field information at the first position-calculating frequency $f_2$ (first detection-magnetic-field components). Here, the second term of the Expression for $V_{m1}{}^1$, that is, the signal detected by the second sense coil 13a at the frequency $f_2$, contains a signal with the frequency $f_2$ of the first alternating magnetic field output from the marker coil 4, as well as a signal with the frequency $f_2$ of the induced magnetic field generated by the magnetic induction coil 5 in response to the first alternating magnetic field from the marker coil 4 (induced magnetic field associated with the first alternating magnetic field).

Here, the signals with the frequencies $f_1$ and $f_2$ of the induced magnetic field associated with the first alternating magnetic field have the characteristic that they differ from each other in the magnitude relationship of intensity with respect to the first alternating magnetic field and that they have substantially the same absolute value of the intensity. Because of this, when the sum of the first term and the second term of each of the Expressions for $V_{m1}^1$ through $V_{m1}^N$, that is, the sum of the single set of first detection-magnetic-field components is calculated, the signals of the induced magnetic field associated with the first alternating magnetic field are cancelled out, whereas the signals of the first alternating magnetic field remain, without being cancelled out.

In this manner, the signals of the induced magnetic field associated with the first alternating magnetic field can be cancelled out by adding the absolute values of the magnetic-field intensity at the single set of first position-calculating frequencies $f_1$ and $f_2$, which are substantially the same frequency away from the resonance frequency $f_0$, with the first position-calculating frequencies $f_1$ and $f_2$ being on either side of the resonance frequency $f_0$. As a result, the signals of the first alternating magnetic field can be extracted easily.

The position/direction analyzing section 22 calculates the position and the direction of the marker coil 4 from $V_{m1}^1$, $V_{m1}^2$, ... $V_{m1}^N$ obtained in the extraction/calculation section 30 (step S147).

Data on the calculated position and direction of the marker coil 4 is sent to the control circuit 28 and displayed on the display device 8 (step S148). Thereafter, the data on the calculated position and direction is accumulated in the second memory 23 (step S149).

Then, it is checked whether or not a command for terminating position detection has been input on the input device 26 (step S150), and if a command has been input, generation of a trigger signal from the trigger generator 31 is terminated to stop the operation of the position detection system 1 (step S151). On the other hand, if no termination command has been input, the flow returns to step S133 to continue position detection.

In this case, for the initial values for iterated arithmetic operations of the positions and directions of the magnetic induction coil 5 and the marker coil 4, the calculation results of the positions and the directions of the magnetic induction coil 5 and the marker coil 4 that have previously been calculated and stored in the second memory 23 are used. By doing so, the convergence time of iterated arithmetic operations can be reduced to calculate the positions and the directions in a shorter period of time.

In this manner, according to the position detection system 1 of this embodiment and a position detection method using the system 1, the signal from the marker coil 4 and the signal from the magnetic induction coil 5 can be completely separated from each other based on position information of both the signals. Consequently, the positions and directions of the marker coil 4 and the magnetic induction coil 5, namely, the positions and directions of the tip of the inserting section 2a of the endoscope apparatus 2 and the capsule medical device 3 disposed in the body cavity, can be obtained precisely.

In this embodiment, because the clock 65 provided in the first capsule medical device 61 and the clock 29 provided in the control section 7 are controlled so as to synchronize with each other, the phase relationship between the first alternating magnetic field to be produced from the marker coil 62 and the second alternating magnetic field to be produced from the magnetic-field generating device 41 can be maintained even if the marker-driving circuit 64 is wirelessly controlled.

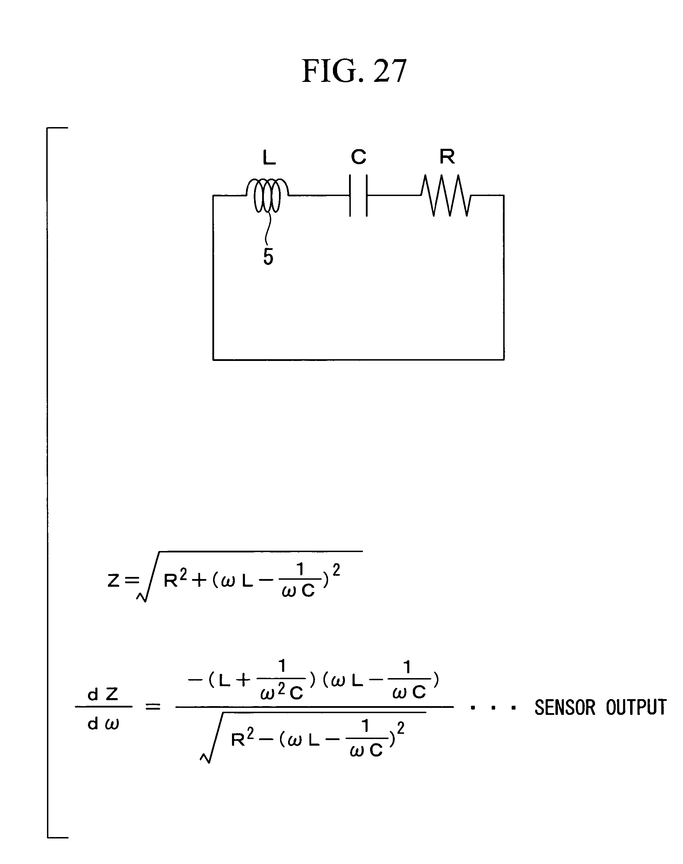

In addition, the position-calculating frequencies $f_1$ and $f_2$ that are set when a magnetic-field waveform according to each of the above-described embodiments is to be generated should preferably be set so as to satisfy the relationship shown in Fig. 27 and Expression 2.

[Expression 2]

$$\frac{-(L+\frac{1}{\omega_1^2 C})(\omega L - \frac{1}{\omega_1 C})}{\sqrt{R^2 - (\omega_1 L - \frac{1}{\omega_1 C})^2}} = \frac{-(L+\frac{1}{\omega_2^2 C})(\omega L - \frac{1}{\omega_2 C})}{\sqrt{R^2 - (\omega_2 L - \frac{1}{\omega_2 C})^2}}$$

where $\omega_1 = 2\pi f_1$, $\omega_2 = 2\pi f_2$, and $\omega_1 < \omega_0 = 2\pi f_0 < \omega_2$ ($f_0$: resonance frequency).

In this case, the intensity signals of the induced magnetic field produced from the magnetic induction coil 5 have the same intensity and opposite polarities at the frequencies $f_1$ and $f_2$. For this reason, the signal component from the magnetic induction coil 5 can be removed while the signal component from the marker coil 4 is retained by adding

$V^{f1-1}$ and $V^{f2-1}$ as-is in actual measurement.

Although the embodiments according to the present invention have been described with reference to the drawings, specific structures are not limited to those of the embodiments. For example, various types of design changes that do not depart from the spirit and scope of the present invention are also included in the present invention.

**Claims**

1. A position detection system comprising:

a first marker that produces, by means of an external power supply, a first alternating magnetic field having a single set of first position-calculating frequencies that are a predetermined frequency away from each other;
a second marker including a magnetic induction coil having as a resonance frequency a substantially central frequency interposed between the single set of first position-calculating frequencies;
a magnetic-field detection section that is disposed outside a working region of the second marker and that detects a magnetic field at the first position-calculating frequencies;
an extracting section that extracts from the magnetic field detected by the magnetic-field detection section the sum of intensities of a single set of first detection-magnetic-field components having the single set of first position-calculating frequencies; and
a position/orientation analyzing section that calculates at least one of a position and a direction of the first marker based on the extracted sum.

2. The position detection system according to claim 1, wherein
the single set of first position-calculating frequencies are frequencies near the resonance frequency,
the extracting section extracts the difference between the intensities of the single set of first detection-magnetic-field components from the magnetic field detected by the magnetic-field detection section; and
the position/orientation analyzing section calculates at least one of a position and a direction of the second marker based on the difference between the intensities.

3. The position detection system according to claim 2 comprising:

a magnetic-field generating unit that is disposed outside the working region of the second marker and that produces a second alternating magnetic field having the single set of first position-calculating frequencies, wherein
the single set of first detection-magnetic-field components are the difference between a magnetic field having the first position-calculating frequencies detected when the first alternating magnetic field is produced and a magnetic field having the first position-calculating frequencies detected before the first alternating magnetic field is produced.

4. The position detection system according to claim 1 comprising:

a magnetic-field generating unit that is disposed outside the working region of the second marker and that produces a second alternating magnetic field having a single set of second position-calculating frequencies that are near the resonance frequency, that differ from the first position-calculating frequencies, and that are a predetermined frequency away from the resonance frequency, with the second position-calculating frequencies and being on either side of the resonance frequency, wherein
the magnetic-field detection section detects a magnetic field at the second position-calculating frequencies,
the extracting section extracts the difference between intensities of a single set of second detection-magnetic-field components having the single set of second position-calculating frequencies from the magnetic field detected by the magnetic-field detection section, and
the position/orientation analyzing section calculates at least one of a position and a direction of the second marker based on the difference between the intensities.

5. The position detection system according to claim 1 comprising:

a magnetic-field generating unit that is disposed outside the working region of the second marker and that produces a second alternating magnetic field having the resonance frequency, wherein
the magnetic-field detection section detects a magnetic field at the resonance frequency,

the extracting section extracts from the magnetic field detected by the magnetic-field detection section a second detection-magnetic-field component that has the resonance frequency and that has a phase shifted by n/2 relative to the phase of the second alternating magnetic field, and

the position/orientation analyzing section calculates at least one of a position and a direction of the second marker based on an intensity of the second detection-magnetic-field component.

6.  The position detection system according to one of claims 1 to 5, wherein
    a resonance circuit including the magnetic induction coil satisfies the following relation at the first position-calculating frequencies.

[Expression 1]

$$\frac{-(L+\frac{1}{\omega_1^2 C})(\omega L - \frac{1}{\omega_1 C})}{\sqrt{R^2 - (\omega_1 L - \frac{1}{\omega_1 C})^2}} = \frac{-(L+\frac{1}{\omega_2^2 C})(\omega L - \frac{1}{\omega_2 C})}{\sqrt{R^2 - (\omega_2 L - \frac{1}{\omega_2 C})^2}}$$

where $\omega_1 = 2\pi f_1$, $\omega_2 = 2\pi f_2$, and $\omega_1 < \omega_0 = 2\pi f_0 < \omega_2$ ($f_0$: resonance frequency).

7.  The position detection system according to one of claims 1 to 6, wherein
    a plurality of the first markers are provided, and
    a plurality of the first position-calculating frequencies differ from one another.

8.  The position detection system according to one of claims 1 to 6, wherein
    the first marker is provided at a front end portion of an endoscope.

9.  The position detection system according to claim 7, wherein
    the plurality of first markers are provided along a longitudinal direction of an inserting section of an endoscope.

10. The position detection system according to claims 1 to 9, wherein
    the second marker is provided in a capsule medical device.

11. The position detection system according to one of claims 2 to 10 further comprising:

    a magnetic-field acting section in the second marker;
    a propulsion-magnetic-field generating unit that produces a propulsion magnetic field acting upon the magnetic-field acting section; and
    a propulsion-magnetic-field control section that controls an intensity and a direction of the propulsion magnetic field based on at least one of the position and the direction of the second marker calculated by the position/orientation analyzing section.

12. A position detection method comprising:

    a magnetic-field generating step of causing a first marker to produce, by means of an external power supply, a first alternating magnetic field having a single set of first position-calculating frequencies that are a predetermined frequency away from each other;
    an induction magnetic-field generating step of causing a second marker having a magnetic induction coil to produce an induced magnetic field in response to the first alternating magnetic field;
    a magnetic-field detecting step of detecting a magnetic field at the first position-calculating frequencies;
    an extracting step of extracting from the detected magnetic field the sum of intensities of a single set of first detection-magnetic-field components having the single set of first position-calculating frequencies; and
    a position/orientation analyzing step of calculating at least one of a position and a direction of the first marker based on the extracted sum.

**13.** The position detection method according to claim 12, wherein
the extracting step includes the step of extracting the difference between the intensities of the single set of first detection-magnetic-field components from the detected magnetic field, and
the position/orientation analyzing step includes the step of calculating at least one of a position and a direction of the second marker based on the extracted difference between the intensities.

**14.** The position detection method according to claim 13, wherein
the magnetic-field generating step includes the step of producing a second alternating magnetic field having the single set of first position-calculating frequencies,
the induction magnetic-field generating step includes the step of causing the second marker to produce an induced magnetic field in response to the second alternating magnetic field, and
the single set of detection-magnetic-field components are the difference between a magnetic field having the first position-calculating frequencies detected when the first alternating magnetic field is produced and a magnetic field having the first position-calculating frequencies detected before the first alternating magnetic field is produced.

**15.** The position detection method according to claim 12, wherein
the magnetic-field generating step includes the step of producing a second alternating magnetic field having a single set of second position-calculating frequencies near the single set of first position-calculating frequencies,
the magnetic-field detecting step includes the step of detecting a magnetic field at the second position-calculating frequencies,
the extracting step includes the step of extracting from the detected magnetic field the difference between intensities of a single set of second detection-magnetic-field components having the single set of second position-calculating frequencies, and
the position/orientation analyzing step includes the step of calculating at least one of a position and a direction of the second marker based on the extracted difference between the intensities.

**16.** The position detection method according to claim 12, wherein
the magnetic-field generating step includes the step of producing a second alternating magnetic field having the resonance frequency,
the magnetic-field detecting step includes the step of detecting a magnetic field at the resonance frequency,
the extracting step includes the step of extracting from the detected magnetic field a second detection-magnetic-field component that has the resonance frequency and that has a phase shifted by n/2 relative to the phase of the second alternating magnetic field, and
the position/orientation analyzing step calculates at least one of a position and a direction of the second marker based on an intensity of the extracted second detection-magnetic-field component.

FIG. 1

# FIG. 2

# FIG. 3

START

INPUT RESONANCE FREQUENCY OF MAGNETIC INDUCTION COIL — S1

SET FREQUENCIES $f_1$ AND $f_2$ — S2

TRANSFER TO WAVEFORM-DATA GENERATOR — S3

CALCULATE MAGNETIC-FIELD WAVEFORM PRODUCED FROM MARKER COIL — S4

TRANSFER WAVEFORM DATA TO MEMORY IN MARKER-DRIVING CIRCUIT — S5

END

# FIG. 4

```
                    ┌──────────┐
                    │  START   │
                    └────┬─────┘
                         │
                         ▼
         ┌──────────────────────────────────┐
         │  INSERT CAPSULE MEDICAL DEVICE    │──── S11
         └──────────────┬───────────────────┘
                        │
                        ▼
         ┌──────────────────────────────────┐
         │     INSTRUCT START OF             │──── S12
         │     ACTUAL MEASUREMENT            │
         └──────────────┬───────────────────┘
                        │
      (D)───────────────┼────────────────────────┐
                        ▼                         │
         ┌──────────────────────────────────┐    │
  S13 ───│   INSTRUCT GENERATION OF          │    │
         │   TRIGGER SIGNAL                  │    │
         └──────────────┬───────────────────┘    │
                        ▼                         │
         ┌──────────────────────────────────┐    │
  S14 ───│   PRODUCE MAGNETIC FIELD          │    │
         │   FROM MARKER COIL                │    │
         └──────────────┬───────────────────┘    │
                        ▼                         │
         ┌──────────────────────────────────┐    │
  S15 ───│  PROCESS MAGNETIC-FIELD SIGNAL    │    │
         │  RECEIVED FROM MARKER COIL        │    │
         └──────────────┬───────────────────┘    │
                        ▼                         │
         ┌──────────────────────────────────┐    │
  S16 ───│   STORE DATA IN FIRST MEMORY      │    │
         └──────────────┬───────────────────┘    │
                        ▼                         │
         ┌──────────────────────────────────┐    │
  S17 ───│   FREQUENCY ANALYSIS              │    │
         │   PROCESSING OF DATA              │    │
         └──────────────┬───────────────────┘    │
                        ▼                         │
              ╱───────────────────╲     NO        │
  S18 ──────╱  ARE DATA             ╲─────────────┘
            ╲  FROM ALL SENSE COILS ╱
             ╲  PROCESSED?         ╱
              ╲───────┬───────────╱
                      │ YES
                      ▼
                    ( B )
```

# FIG. 5

B

S19 — SAVE ONLY MAGNETIC-FIELD
INFORMATION AT FREQUENCY
$f_0$ IN THIRD MEMORY

S20 — ARE DATA
FROM ALL SENSE COILS
PROCESSED? — NO

YES

S21 — EXTRACT SIGNAL FOR POSITION
CALCULATION OF MAGNETIC
INDUCTION COIL

S22 — CALCULATE POSITION AND
DIRECTION OF MAGNETIC
INDUCTION COIL

S23 — DISPLAY POSITION AND
DIRECTION DATA

S24 — STORE POSITION AND
DIRECTION DATA

S25 — EXTRACT SIGNAL FOR POSITION
CALCULATION OF MARKER COIL

C

# FIG. 6

```
                    ( C )
                      |
                      v
         +---------------------------+
         |  CALCULATE POSITION AND   |---- S26
         |  DIRECTION OF MARKER COIL |
         +---------------------------+
                      |
                      v
         +---------------------------+
         |    DISPLAY POSITION AND   |---- S27
         |      DIRECTION DATA       |
         +---------------------------+
                      |
                      v
         +---------------------------+
         |     STORE POSITION AND    |---- S28
         |      DIRECTION DATA       |
         +---------------------------+
                      |
                      v          S29
                  /----------\         NO
                 <  IS STOP   >-------------> ( D )
                 < COMMAND    >
                  \ ENTERED? /
                   \--------/
                      | YES
                      v
              +----------------+
              |  STOP SYSTEM   |---- S30
              +----------------+
                      |
                      v
                (   END   )
```

FIG. 7

EP 2 149 327 A1

# FIG. 8

EP 2 149 327 A1

FIG. 9

DRIVING FREQUENCIES $(f_1, f_2)$

DRIVING FREQUENCIES $(f_1, f_2)$

CONNECTED BY WIRES

EP 2 149 327 A1

FIG. 10

FIG. 11

```
        ┌─────────────┐
        │    START    │
        └──────┬──────┘
               │
               ▼
┌──────────────────────────────┐
│     INSTRUCT CALIBRATION      │─────S31
└──────────────┬───────────────┘
               │
               ▼
┌──────────────────────────────┐
│    INSTRUCT GENERATION OF     │─────S32
│       TRIGGER SIGNAL          │
└──────────────┬───────────────┘
               │
               ▼
┌──────────────────────────────┐
│     PRODUCE MAGNETIC FIELD    │
│     FROM MAGNETIC-FIELD       │─────S33
│      GENERATING DEVICE        │
└──────────────┬───────────────┘
               │
               ▼
┌──────────────────────────────┐
│  PROCESS MAGNETIC-FIELD SIGNAL│
│   RECEIVED FROM MAGNETIC-FIELD│─────S34
│      GENERATING DEVICE        │
└──────────────┬───────────────┘
               │
               ▼
┌──────────────────────────────┐
│   STORE DATA IN FIRST MEMORY  │─────S35
└──────────────┬───────────────┘
               │
               ▼
┌──────────────────────────────┐
│      FREQUENCY ANALYSIS       │─────S36
│      PROCESSING OF DATA       │
└──────────────┬───────────────┘
               │
               ▼
┌──────────────────────────────┐
│  WRITE INTO MEMORY MAGNETIC-FIELD│
│   INFORMATION AT FREQUENCIES  │─────S37
│  f₁ AND f₂ AS CALIBRATION VALUE│
└──────────────┬───────────────┘
               │
               ▼
┌──────────────────────────────┐
│     PERFORM CORRECTION OF     │
│  EQUALIZING SIGNAL INTENSITIES│─────S38
│      AND STORE IN MEMORY      │
└──────────────┬───────────────┘
               │
               ▼
        ┌─────────────┐
        │     END     │
        └─────────────┘
```

In step S37: WRITE INTO MEMORY MAGNETIC-FIELD INFORMATION AT FREQUENCIES $f_1$ AND $f_2$ AS CALIBRATION VALUE

FIG. 12

START

INSERT CAPSULE MEDICAL DEVICE
AND INSERTING SECTION OF
ENDOSCOPE APPARATUS — S41

INSTRUCT START OF ACTUAL
MEASUREMENT — S42

H

S43 — INSTRUCT GENERATION OF
TRIGGER SIGNAL

S44 — PRODUCE MAGNETIC FIELD
FROM MARKER COIL

S45 — PRODUCE MAGNETIC FIELD
FROM MAGNETIC-FIELD
GENERATING DEVICE

S46 — PROCESS MAGNETIC-FIELD SIGNAL
RECEIVED FROM MAGNETIC-FIELD
GENERATING DEVICE

S47 — STORE DATA IN FIRST MEMORY

S48 — FREQUENCY ANALYSIS
PROCESSING OF DATA

S49 — ARE DATA
FROM ALL SENSE COILS
PROCESSED?

NO

YES

F

# FIG. 13

(F)

S50 — SAVE IN THIRD MEMORY ONLY MAGNETIC-FIELD INFORMATION AT FREQUENCIES $f_1$ AND $f_2$ FROM MARKER COIL AND MAGNETIC-FIELD GENERATING DEVICE

S51 — ARE DATA FROM ALL SENSE COILS PROCESSED? — NO

YES

S52 — EXTRACT SIGNAL FOR POSITION CALCULATION OF MAGNETIC INDUCTION COIL

S53 — CALCULATE POSITION AND DIRECTION OF MAGNETIC INDUCTION COIL

S54 — DISPLAY POSITION AND DIRECTION DATA

S55 — STORE POSITION AND DIRECTION DATA

(G)

# FIG. 14

G

S56 — EXTRACT SIGNAL FOR POSITION
CALCULATION OF MARKER COIL

S57 — CALCULATE POSITION AND
DIRECTION OF MARKER COIL

S58 — DISPLAY POSITION AND DIRECTION DATA

S59 — STORE POSITION AND DIRECTION DATA

S60 — IS STOP
COMMAND ENTERED?          NO → H

YES

S61 — STOP SYSTEM

END

FIG. 15

DRIVING
FREQUENCIES
(f3, f4)

DRIVING
FREQUENCIES (f1, f2)

CONNECTED
BY WIRES

EP 2 149 327 A1

## FIG. 16

START

INSTRUCT CALIBRATION — S71

INSTRUCT GENERATION OF
TRIGGER SIGNAL — S72

PRODUCE MAGNETIC FIELD
FROM MAGNETIC-FIELD
GENERATING DEVICE — S73

PROCESS MAGNETIC-FIELD SIGNAL
RECEIVED FROM MAGNETIC-FIELD
GENERATING DEVICE — S74

STORE DATA IN FIRST MEMORY — S75

FREQUENCY ANALYSIS
PROCESSING OF DATA — S76

WRITE INTO MEMORY MAGNETIC-FIELD
INFORMATION AT FREQUENCIES
$f_3$ AND $f_4$ AS CALIBRATION VALUE — S77

PERFORM CORRECTION OF
EQUALIZING SIGNAL INTENSITIES
AND STORE IN MEMORY — S78

END

FIG. 17

START

S81 — INSERT CAPSULE MEDICAL DEVICE AND INSERTING SECTION OF ENDOSCOPE APPARATUS

S82 — INSTRUCT START OF ACTUAL MEASUREMENT

(H)

S83 — INSTRUCT GENERATION OF TRIGGER SIGNAL

S84 — PRODUCE MAGNETIC FIELD FROM MARKER COIL

S85 — PRODUCE MAGNETIC FIELD FROM MAGNETIC-FIELD GENERATING DEVICE

S86 — PROCESS MAGNETIC-FIELD SIGNAL RECEIVED FROM MAGNETIC-FIELD GENERATING DEVICE

S87 — STORE DATA IN FIRST MEMORY

S88 — FREQUENCY ANALYSIS PROCESSING OF DATA

S89 — ARE DATA FROM ALL SENSE COILS PROCESSED?

NO

YES

(F)

# FIG. 18

```
                    ( F )
                      │
                      ▼◄──────────────────┐
┌──────────────────────────────────────┐  │
│        SAVE IN THIRD MEMORY ONLY       │  │
│  MAGNETIC-FIELD INFORMATION AT FREQUENCIES │  │
│      f₁ AND f₂ FROM MARKER COIL AND    │  │
│      MAGNETIC-FIELD GENERATING DEVICE  │  │
└──────────────────────────────────────┘  │
```

S90 — SAVE IN THIRD MEMORY ONLY MAGNETIC-FIELD INFORMATION AT FREQUENCIES $f_1$ AND $f_2$ FROM MARKER COIL AND MAGNETIC-FIELD GENERATING DEVICE

S91 — ARE DATA FROM ALL SENSE COILS PROCESSED?   NO

YES

S92 — EXTRACT SIGNAL FOR POSITION CALCULATION OF MAGNETIC INDUCTION COIL

S93 — CALCULATE POSITION AND DIRECTION OF MAGNETIC INDUCTION COIL

S94 — DISPLAY POSITION AND DIRECTION DATA

S95 — STORE POSITION AND DIRECTION DATA

( G )

# FIG. 19

G

S96 — EXTRACT SIGNAL FOR POSITION
CALCULATION OF MARKER COIL

S97 — CALCULATE POSITION AND
DIRECTION OF MARKER COIL

S98 — DISPLAY POSITION AND DIRECTION DATA

S99 — STORE POSITION AND DIRECTION DATA

S100 — IS STOP
COMMAND ENTERED? — NO → H

YES

S101 — STOP SYSTEM

END

FIG. 20

EP 2 149 327 A1

FIG. 21

# FIG. 22

```
          ┌─────────────┐
          │    START    │
          └─────────────┘
                 │
                 ▼
   ┌──────────────────────────────┐
   │  INPUT RESONANCE FREQUENCY OF │ ── S111
   │    MAGNETIC INDUCTION COIL    │
   └──────────────────────────────┘
                 │
                 ▼
   ┌──────────────────────────────┐
   │  SET FREQUENCIES f0, f1, AND f2 OF │ ── S112
   │  FIRST AND SECOND ALTERNATING │
   │        MAGNETIC FIELDS        │
   └──────────────────────────────┘
                 │
                 ▼
   ┌──────────────────────────────┐
   │ TRANSFER TO WAVEFORM-DATA GENERATOR │ ── S113
   └──────────────────────────────┘
                 │
                 ▼
   ┌──────────────────────────────┐
   │  CALCULATE MAGNETIC-FIELD WAVEFORM │ ── S114
   │     PRODUCED FROM MARKER COIL │
   └──────────────────────────────┘
                 │
                 ▼
   ┌──────────────────────────────┐
   │  CALCULATE MAGNETIC-FIELD WAVEFORM │ ── S115
   │     PRODUCED FROM MAGNETIC-FIELD │
   │        GENERATING DEVICE      │
   └──────────────────────────────┘
                 │
                 ▼
   ┌──────────────────────────────┐
   │ TRANSFER AND STORE WAVEFORM DATA IN │ ── S116
   │  MEMORY OF MARKER-DRIVING CIRCUIT │
   └──────────────────────────────┘
                 │
                 ▼
   ┌──────────────────────────────┐
   │ TRANSFER AND STORE WAVEFORM DATA IN │ ── S117
   │ MEMORY OF MAGNETIC-FIELD-GENERATING │
   │      -DEVICE DRIVING CIRCUIT  │
   └──────────────────────────────┘
                 │
                 ▼
          ┌─────────────┐
          │     END     │
          └─────────────┘
```

Input resonance frequency of magnetic induction coil — S111

Set frequencies $f_0$, $f_1$, and $f_2$ of first and second alternating magnetic fields — S112

Transfer to waveform-data generator — S113

Calculate magnetic-field waveform produced from marker coil — S114

Calculate magnetic-field waveform produced from magnetic-field generating device — S115

Transfer and store waveform data in memory of marker-driving circuit — S116

Transfer and store waveform data in memory of magnetic-field-generating-device driving circuit — S117

# FIG. 23

START

INSTRUCT SETTING OF READ-OUT TIMING — S121

INSTRUCT GENERATION OF TRIGGER SIGNAL — S122

PRODUCE MAGNETIC FIELD FROM MAGNETIC-FIELD GENERATING DEVICE — S123

PROCESS MAGNETIC-FIELD SIGNAL RECEIVED FROM MAGNETIC-FIELD GENERATING DEVICE — S124

STORE DATA IN FIRST MEMORY — S125

FREQUENCY ANALYSIS PROCESSING OF DATA — S126

WRITE INTO MEMORY ONLY MAGNETIC-FIELD INFORMATION AT FREQUENCY $f_0$ — S127

STORE VALUE OF IMAGINARY PART OF INFORMATION WRITTEN INTO MEMORY — S128

SEND COMMAND FOR DELAYING READ-OUT TIMING BY ONE CLOCK — S129

SET READ-OUT TIMING THAT CAUSES IMAGINARY PART TO BECOME CLOSEST TO ZERO — S130

END

FIG. 24

START

S131 — INSERT CAPSULE MEDICAL DEVICE

S132 — INSTRUCT START OF ACTUAL MEASUREMENT

(L)

S133 — INSTRUCT GENERATION OF TRIGGER SIGNAL

S134 — PRODUCE MAGNETIC FIELD
FROM MARKER COIL

S135 — PRODUCE MAGNETIC FIELD FROM
MAGNETIC-FIELD GENERATING DEVICE

S136 — PROCESS RECEIVED
MAGNETIC-FIELD SIGNAL

S137 — STORE DATA IN FIRST MEMORY

S138 — FREQUENCY ANALYSIS PROCESSING OF DATA

S139
ARE DATA
FROM ALL SENSE COILS
PROCESSED?

NO

YES

J

# FIG. 25

(J)

S140 — EXTRACT MAGNETIC-FIELD INFORMATION AT FREQUENCIES $f_1$ AND $f_2$ AND SAVE IN THIRD MEMORY IN ASSOCIATION WITH FREQUENCIES $f_1$ AND $f_2$

S141 — EXTRACT MAGNETIC-FIELD INFORMATION AT FREQUENCY $f_0$ AND SAVE IN THIRD MEMORY

S142 — ARE DATA FROM ALL SENSE COILS PROCESSED? — NO

YES

S143 — XTRACT SIGNAL FOR POSITION CALCULATION OF MAGNETIC INDUCTION COIL

S144 — CALCULATE POSITION AND DIRECTION OF MAGNETIC INDUCTION COIL

S145 — DISPLAY POSITION AND DIRECTION DATA

S146 — STORE POSITION AND DIRECTION DATA

(K)

# FIG. 26

```
                    ( K )
                      │
                      ▼
   ┌──────────────────────────────────────┐
   │    CALCULATE POSITION AND            │──── S147
   │    DIRECTION OF MARKER COIL          │
   └──────────────────────────────────────┘
                      │
                      ▼
   ┌──────────────────────────────────────┐
   │  DISPLAY POSITION AND DIRECTION DATA │──── S148
   └──────────────────────────────────────┘
                      │
                      ▼
   ┌──────────────────────────────────────┐
   │  STORE POSITION AND DIRECTION DATA   │──── S149
   └──────────────────────────────────────┘
      S150              │
         ╲              ▼
          ◇─────────────────────────◇    NO
          ╱     IS STOP              ╲ ──────── ( L )
          ╲ COMMAND ENTERED?         ╱
           ◇───────────────────────◇
                      │ YES
                      ▼
          ┌──────────────────────┐
          │     STOP SYSTEM      │──── S151
          └──────────────────────┘
                      │
                      ▼
             (    END    )
```

# FIG. 27

$$Z = \sqrt{R^2 + (\omega L - \frac{1}{\omega C})^2}$$

$$\frac{dZ}{d\omega} = \frac{-(L + \frac{1}{\omega^2 C})(\omega L - \frac{1}{\omega C})}{\sqrt{R^2 - (\omega L - \frac{1}{\omega C})^2}} \quad \cdots \quad \text{SENSOR OUTPUT}$$

## INTERNATIONAL SEARCH REPORT

International application No.

PCT/JP2008/057961

A. CLASSIFICATION OF SUBJECT MATTER
*A61B1/00*(2006.01)i, *G01B7/00*(2006.01)i

According to International Patent Classification (IPC) or to both national classification and IPC

B. FIELDS SEARCHED

Minimum documentation searched (classification system followed by classification symbols)
A61B1/00-1/32, A61B5/06, A61B5/07, G01B7/00

Documentation searched other than minimum documentation to the extent that such documents are included in the fields searched
| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
JSTPlus(JDreamII), JMEDPlus(JDreamII), JST7580(JDreamII)

C. DOCUMENTS CONSIDERED TO BE RELEVANT

| Category* | Citation of document, with indication, where appropriate, of the relevant passages | Relevant to claim No. |
|---|---|---|
| A | JP 2000-81303 A (Olympus Optical Co., Ltd.), 21 March, 2000 (21.03.00), Full text; all drawings & US 6511417 B1 | 1-16 |
| A | Yuki TOKUNAGA et al., "LC Kyoshingata Jiki Marker o Mochiita Koseido Ichi Kenshutsu System", Journal of Magnetics Society of Japan, 2005, Vol.29, No.2, pages 153 to 156 | 1-16 |
| A | JP 2003-290129 A (Olympus Optical Co., Ltd.), 14 October, 2003 (14.10.03), Full text; all drawings (Family: none) | 1-16 |

☐ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search 14 May, 2008 (14.05.08) | Date of mailing of the international search report 27 May, 2008 (27.05.08) |
|---|---|
| Name and mailing address of the ISA/ Japanese Patent Office | Authorized officer |
| Facsimile No. | Telephone No. |

Form PCT/ISA/210 (second sheet) (April 2007)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000081303 A

**Non-patent literature cited in the description**

- **Tokunaga.** Precision Position-detecting System Using an LC Resonant Magnetic Marker. *Journal of the Magnetics Society of Japan,* 2005, vol. 29 (2), 153-156